(19) Europäisches Patentamt
European Patent Office
Office européen des brevets

(11) Publication number: **0 191 869**
**A1**

(12) # EUROPEAN PATENT APPLICATION

published in accordance with Art. 158(3) EPC

(21) Application number: 85904145.1

(22) Date of filing: 16.08.85

Data of the international application taken as a basis:

(86) International application number: PCT/JP85/00459

(87) International publication number: WO86/01226 (27.02.86 86/05)

(51) Int. Cl.[4]: C 12 N 15/00
C 12 N 1/20, C 07 K 7/36
C 12 P 21/02
//(C12N1/20, C12R1:19),
(C12P21/02, C12R1:19)

(30) Priority: 17.08.84 JP 170492/84
11.05.85 JP 98891/85
28.05.85 JP 113254/85
18.06.85 JP 130815/85

(43) Date of publication of application:
27.08.86 Bulletin 86/35

(84) Designated Contracting States:
AT BE CH DE FR GB IT LI LU NL SE

(71) Applicant: SAGAMI CHEMICAL RESEARCH CENTER
4-5, Marunouchi 1-chome
Chiyoda-ku, Tokyo, 100(JP)

(71) Applicant: CENTRAL GLASS COMPANY, LIMITED
5253, Oaza Okiube Ube-shi
Yamaguchi 755(JP)

(71) Applicant: HODOGAYA CHEMICAL COMPANY, LIMITED
4-2, Toranomon 1-chome Minato-ku
Tokyo 105(JP)

(71) Applicant: NIPPON SODA CO., LTD.
2-1, Ohte-machi 2-chome
Chiyoda-ku, Tokyo, 100(JP)

(71) Applicant: NISSAN CHEMICAL INDUSTRIES LTD.
3-7-1, Kanda Nishiki-cho
Chiyoda-ku Tokyo(JP)

(71) Applicant: TOYO SODA MANUFACTURING CO., LTD.
No. 4560, Oaza-tonda Shin-nanyo-shi
Yamaguchi-ken(JP)

(72) Inventor: OMORI, Muneki
2-5-9, Zoshigaya Toshima-ku
Tokyo 171(JP)

(72) Inventor: MIKI, Tetsuzo
2-1-A-908, Shinshinden Abiko-shi
Chiba 270-11(JP)

(72) Inventor: NARUSHIMA, Hiroyuki
4-4-1, Nishionuma Sagamihara-shi
Kanagawa 229(JP)

(72) Inventor: IKARI, Takaomi
4-4-1, Nishionuma Sagamihara-shi
Kanagawa 229(JP)

(72) Inventor: SAITO, Akemi
3-6-1-303, Susukino Modori-ku
Yokohama-shi Kanagawa 227(JP)

(72) Inventor: IKUSHIMA, Noriko
17-6, Asahi-cho Sagamihara-shi
Kanagawa 228(JP)

(72) Inventor: MATSUMOTO, Reiko
2621, Asamizodai Sagamihara-shi
Kanagawa 228(JP)

(72) Inventor: WATANABE, Kazuo
1-9-1, Minamidai Sagamihara-shi
Kanagawa 228(JP)

(74) Representative: Patentanwälte Grünecker, Kinkeldey, Stockmair & Partner
Maximilianstrasse 58
D-8000 München 22(DE)

(54) **FISH CALCITONIN DERIVATIVE, PROCESS FOR ITS PREPARATION, AND GENE SYSTEM THEREFOR.**

(57) This derivative comprises 33 amino acids wherein glycine is added to the C-terminal of fish calcitonin composed of 32 amino acids. This derivative itself has a biological activity and can be converted into natural fish calcitonin composed of 32 amino acids wherein proline in the C-terminal is amidated, by treating it in a conventional manner.

./...

EP 0 191 869 A1

Croydon Printing Company Ltd

```
          1   2   3   4   5   6   7   8   9   10  11  12  13  14  15  16  17  18  19
      Met Cys Ser Asn Leu Ser Thr Cys Val Leu Gly Lys Leu Ser Gln Glu Leu His Lys Leu
          U-11 (19)               U-2 (17)                U-31 (15)           U-41 (15)
     GTT AAC ATG TGC TCC AAT CTC TCT ACT TGC GTT CTG GGG AAG TTG AGT CAG GAA TTA CAT AAG CTG

ACGT CAA TTG TAC ACG AGG TTA GAG AGA TGA ACG CAA GAC CCC TTC AAC TCA GTC CTT AAT G
     L-11 (15)                L-2 (17)                L-31 (15)           L-41 (15)


17  18  19  20  21  22  23  24  25  26  27  28  29  30  31  32  33
His Lys Leu Gln Thr Tyr Pro Arg Thr Asn Thr Gly Ser Gly Thr Pro Gly stp stp
                    U-5 (15)                U-6 (17)                U-7 (18)
            CAA ACT TAC CCG CGT ACC AAC ACT GGT TCT GGT ACA CCT GGT TAA TAG AT

 TA TTC GAC GTT TGA ATG GGC GCA TGG TTG TGA CCA AGA CCA TGT GGA CCA ATT ATC TAG C
        L-5 (16)                L-6 (17)                L-7 (14)          L-8 (13)


                                                         U-7' (15)
                                                  T ACA CCT TAA TAG AT
                        Without Gly (33) codon
                                                      ATT ATC TAG C
                                                        L-8' (10)
```

Fig. 1

# DESCRIPTION

## TITLE OF THE INVENTION

Fish Calcitonin Derivative, Process for Preparation Thereof, and Gene System Therefor

## TECHNICAL FIELD

The present invention relates to novel fish calcitonin derivatives having calcitonin activities, a genetic engineering process for the preparation thereof, and gene systems for the production of fish calcitonin and the derivatives thereof.

## BACKGROUND ART

Calcitonin has been found to be effective in the treatment of hypercalcemia and Paget's disease. It has also been verified that calcitonin inhibits bone resorption and promotes bone neoformation. In addition, a therapeutic effect therefrom on senile osteoporosis is anticipated. Calcitonins have been isolated from swine, cattle, human beings, rats, chickens, salmon, and eels.

A comparison of the physiological activities in the blood of calcitonins derived from various animal species has shown that calcitonin obtained from the fish ultimobranchial body is about 30 times more active than that derived from the mammalian thyroid gland, and that the blood level thereof lasts longer. For example, in their reports, H. Copp et al (Calcitonin, Proc. 2nd Int. Symp, London), Heineman 281 (1981), attributed this to the fact that, compared with other calcitonins, the salmon calcitonin is not readily inactivated in the blood. The eel calcitonin in particular is not only very active but is also very stable *in vitro* in the serum or in extracts from such organs as the liver and kidney. Accordingly it is particularly advantageous to use fish calcitonin as a pharmaceutical.

The structures of calcitonins isolated from swine, cattle, human beings, rats, chickens, salmon, and eels have been elucidated. For example, the salmon calcitonin and eel calcitonin referred to as fish calcitonin in the present invention are polypeptides each consisting of 32 amino acids, and the carboxyl group of the proline, which is the C-end amino acid, is amidated. Such an amide structure has been believed to be necessary for the calcitonin activity (see, for example, S. Guttman, Excerpta Medica Int. Cong. Ser. 540, 11, 1981). The present inventors, however, have found that although it is a derivative of the fish calcitonin without such an amide structure, it exhibits a calcitonin activity when administered to mammals.

The calcitonins commercially available today for therapeutic purposes are derived from swine, salmon, and eels, but are obtained by extraction from organisms or by chemical synthesis. However, their production is limited and the costs thereof are exorbitant. Therefore, the development of a novel process for preparing fish calcitonin and a derivative thereof economically and in large quantities is strongly desired.

For this purpose, a process employing gene manipulation techniques is most preferred. Japanese Unexamined Patent Publication No. 58-203953 describes a chemically synthesized gene for human calcitonin and its expression in E. coli; a PCT-National Publication in Japan No. 58-501121 describes a gene for a human calcitonin precursor; another PCT-National Publication in Japan No. 59-501095 describes a chemically synthesized gene for human calcitonin and its expression; and yet another PCT-National Publication in Japan No. 59-501243 describes a gene for naturally occurring human calcitonin and its expression.

The synthesis of a gene for fish calcitonin or a gene for a fish calcitonin derivative, and the expression thereof in E. coli have not been reported as yet.

DISCLOSURE OF THE INVENTION

The present invention provides novel fish calcitonin derivatives. These derivatives are polypeptides consisting of 33 amino acids having an additional glycine preceded by the proline at the C-terminal of the fish calcitonin polypeptide consisting of 32 amino acids. The derivatives exhibit significant calcitonin activities when administered to mammals. In addition, the derivatives may be converted to naturally-occurring type fish calcitonin consisting of 32 amino acids with the amidated proline at the C-terminal by in vitro treatment conducted in accordance with well-known methods, K. Breddam, F. Widmer, and J.T. Johonsen, Carlsberg Res. Commun. Vol 45, 237-247 (1980); and ibid Vol 45, 361-367 (1980).

The present invention also provides useful systems of genes for the preparation of the fish calcitonin derivatives. These gene systems include structural genes coding for the fish calcitonin derivatives, DNA fragments in which the structural gene is connected to a structural gene for another protein, plasmids containing the structural gene or the DNA fragment, and microorganisms containing the plasmid. In addition, the present invention provides a process for the preparation of this gene system.

Furthermore, the present invention provides a process for preparing fish calcitonins using the gene systems. This process is characterized by culturing E. coli transformed by a plasmid which contains a gene coding for the fish calcitonin derivatives and a gene coding for another protein, and is capable of expressing these genes in E. coli to produce a fused protein composed of the fish calcitonin derivative and another protein, recovering the fused protein, cutting the recovered fused protein to liberate the fish calcitonin derivative, and obtaining the derivative. This process makes it possible to prepare fish calcitonin derivatives economically and on an industrial scale.

BRIEF DESCRIPTION OF THE DRAWINGS

Figure 1 shows the DNA sequences of the genes coding for the salmon calcitonin and its derivative, respectively, which are designed in accordance with the present invention. These include the structural gene and the peripheral area and possess PstI cohesive end on the 5'-side of the coding chain;

Fig. 2 similarly shows the DNA sequence having a recognition site for an HpaI restriction enzyme at the 5'-end of the code chain;

Fig. 3 shows the combination of subblocks used in the synthesis of the salmon calcitonin gene (CT1) and the salmon calcitonin derivative gene (CT2) wherein black points represent the enzymatic addition of the phosphate group;

Fig. 4 is a radioautogram verifying the formation of the DNA fragment containing the salmon calcitonin derivative gene (CT2) from the subblocks 3-5 and 3-7;

Fig. 5 is a flow sheet of the process for constructing the recombinant plasmid pSCT1 or pSCT2 from the starting plasmid pHT2 and the fragment containing the gene CT1 or the fragment containing the gene CT2, respectively, according to the present invention;

Fig. 6 is a diagram showing the construction of the plasmids pLMC2 and pPMC2 of the present invention;

Fig. 7 is a diagram showing the construction of the plasmids pTMC2, pTMC12, pTMC22, and pTMC32 of the present invention;

Fig. 8 shows the construction of the plasmid pLMC2 (A) and the base sequence (B) at the site connecting the metapyrocatechase (C230) code region to the salmon calcitonin (CT) code region;

Fig. 9 shows the construction of the plasmid pPMC2;

Fig. 10 shows the construction of the plasmid pSCT12;

Fig. 11 shows the construction of the plasmid pUCT22;

Fig. 12 shows the construction of the plasmid pTMC2 (A) and the base sequence (B) at the site connecting the metapyrocatechase (C230) code region to the salmon calcitonin derivative (CT) code region;

Fig. 13 shows the construction of the plasmid pTMC22;

Fig. 14 shows the construction of the plasmid pTMC32;

Fig. 15 shows the construction of the plasmid pHT31;

Fig. 16 shows the construction of the plasmid pKTL1;

Fig. 17 shows the relationship between the nucleotide sequence for the salmon calcitonin derivative gene and the amino acid sequence of the salmon calcitonin derivative, the nucleotide sequence for the eel calcitonin derivative gene and the amino acid sequence of the eel calcitonin derivative, the primer oligonucleotide, and the probe oligonucleotide;

Fig. 18 (A) shows the construction of the plasmid pCTM4 derived from the phage CTM41 and the plasmid pTCCM1, and (B) shows the nucleotide sequence at the site connecting the pTCCM1-derived DNA fragment to the CTM41-derived DNA fragment;

Fig. 19 (A) shows an elution of the fused protein in Sephadex G-75 column chromatography, and (B) shows gel patterns obtained by SDS polyacrylamide gel electrophoresis of individual fractions;

Fig. 20 shows the $C_4$ reverse-phase HPLC pattern of the CNBr-treated product using PR304 (A), and the HPLC of the partially purified fractions using a TSK-G2000SW column (B);

Fig. 21 shows the process for the purification of the fused protein produced by <u>E. coli</u> containing the plasmid pCTM4;

Fig. 22 shows the profile of the purification of the eel calcitonin derivative, by C18 column chromatography;

Fig. 23 shows the profile of the purification of the fraction eluted from the C18 column, by gel filtration using a TSK-G2000SW column;

Fig. 24 shows the profile of the purification by reversed-phase HPLC using a RP-304 column; and,

Fig. 25 shows an analysis (A) of the final fraction by PR304 reverse phase HPLC and an analysis (B) of the same by a TSK-G2000SW gel.

BEST MODE FOR CARRYING OUT THE INVENTION

A. Fish Calcitonin Derivative

The fish calcitonin derivative of the invention consists of 32 amino acids of the fish calcitonin and an additional glycine added at the C-terminal thereof, and is represented by the following amino acid sequence (I):

```
Cys Ser Asn Leu Ser Thr Cys Val Leu Gly
Lys Leu Ser Gln Glu Leu His Lys Leu Gln
Thr Tyr Pro Arg Thr  X   Y  Gly  Z  Gly
Thr Pro Gly
```

wherein X represents Asn or Asp, Y represents Thr or Val, and Z represents Ser or Ala.

For example, the salmon calcitonin derivative has the following amino acid sequence (II):

```
 1   2   3   4   5   6   7   8   9   10
Cys Ser Asn Leu Ser Thr Cys Val Leu Gly

 11  12  13  14  15  16  17  18  19  20
Lys Leu Ser Gln Glu Leu His Lys Leu Gln

 21  22  23  24  25  26  27  28  29  30
Thr Tyr Pro Arg Thr Asn Thr Gly Ser Gly

 31  32  33
Thr Pro Gly
```

and consists of 32 amino acids of the natural salmon calcitonin and an additional glycine added at the C-terminal.

The eel calcitonin derivative has the following amino acid sequence (III):

```
 1   2   3   4   5   6   7   8   9   10
Cys Ser Asn Leu Ser Thr Cys Val Leu Gly
```

| | | | | | | | | | |
|---|---|---|---|---|---|---|---|---|---|
| 11 | 12 | 13 | 14 | 15 | 16 | 17 | 18 | 19 | 20 |
| Lys | Leu | Ser | Gln | Glu | Leu | His | Lys | Leu | Gln |
| 21 | 22 | 23 | 24 | 25 | 26 | 27 | 28 | 29 | 30 |
| Thr | Tyr | Pro | Arg | Thr | Asp | Val | Gly | Ala | Gly |
| 31 | 32 | 33 | | | | | | | |
| Thr | Pro | Gly | | | | | | | |

and consists of 32 amino acids of the natural eel calcitonin and an additional glycine added at the C-end.

The fish calcitonin derivative of the present invention is, however, not to be construed as being limited to the salmon calcitonin derivative having the amino acid sequence (II) and the eel calcitonin derivative having the amino acid sequence (III) above. Any calcitonin derivatives comprising any particular combinations of amino acid residues X, Y, and Z in the amino acid sequence shown in the general formula (I) above are considered to be within the scope of the present invention.

B. Gene System for Preparing the Fish Calcitonin Derivative

In order to prepare a relatively low molecular peptide such as calcitonin by genetic engineering techniques using E. coli, it is generally considered advisable to produce a fused protein by using a gene coding for the fused protein consisting of an intended peptide and another protein, and to then cut it to give the intended peptide.

The process of the present invention therefore comprises first connecting a gene coding for the fish calcitonin derivative to a gene coding for another protein such as metapyrocatechase or a part thereof, constructing a plasmid containing such a gene for expression in E. coli, and then culturing the E. coli transformed by this plasmid to give a fused protein. Since the fused protein mainly accumulates in cells of E. coli, the cells are first separated from the culture broth, the fused protein recovered from the cells, the fused protein is cut to liberate the intended fish calcitonin derivative, and finally, the crude derivative is recovered and purified.

1. Construction of the Gene System for the Salmon Calcitonin Derivative

(1) Synthesis of the Structural Gene for the Salmon Calcitonin Derivative

A coding region coding for 5 recurring leucines (Leu) is present in the structural gene coding for the salmon calcitonin derivative, and it was thought to be difficult to synthesize a double-stranded polynucleotide having such a high homology (Refer to J. Windus et al, Nucleic Acids Research, 10,6639, 1982).

Because of degeneracy of the genetic code, a multiplicity of nucleotide sequences is present for an amino acid sequence. Therefore, the use of a trinucleotide codon which is frequently used or is preferably used for a well-expressed protein in *E. coli* is generally recommended. In the case of the salmon calcitonin derivative including a region wherein the same repeated amino acids are present, however, selection of a specific nucleotide sequence does not permit the synthesis of the desired gene. In the present invention, triplet codons frequently used in *E. coli* for respective amino acids are therefore first selected on the basis of the amino acid sequence of the salmon calcitonin or of its derivative to tentatively design a double-stranded DNA sequence. Next a translation initiation codon, i.e., a methionine codon ATG/TAC, is added at the 5'-end of the sequence, and then one, or preferably more than one, translation termination codons are added at the 3'-end. Additionally, appropriate restriction enzyme ends or restriction enzyme recognition sequences are added at both ends of the DNA sequence. The code chain of the thus postulated double-stranded DNA sequence is divided into appropriate lengths, providing, for example, oligonucleotides such as U-1 to L-7, and then the anticode chain is divided into appropriate lengths so that they span the oligonucleotide connecting point of the code chain, providing oligonucleotides such as L-1 to L-8. Free energy of hybridization is evaluated for the oligonucleotide binding sites thus designed, and free energy of cross-hybridization between the oligonucleotides constituting the double-stranded nucleotide sequence thus selected is evaluated. For portions where the cross-hybridization is judged to be preferential over the hybridization by the comparison of the two values, triplet codons for amino acids are replaced by other triplet codons for the same amino acids to modify the double-stranded nucleotide. Such modifications are repeated to establish a preferred nucleotide sequence. The value obtained using the method for estimating the degree of hybridization for the attenuator structure in the RNA nucleotide sequence (see, for example, I. Tinoco Jr. et al, Nature New Biology 246 40, 1973) may substitute for the value of free energy of the hybridization.

Specifically, oligonucleotides U3 and U4 are modified to U31 and U41. These oligonucleotides have the following relationship:

| 10 | 11 | 12 | 13 | 14 | 15 | 16 | 17 | 18 | 19 |
|---|---|---|---|---|---|---|---|---|---|
| Gly | Lys | Leu | Ser | Gln | Glu | Leu | His | Lys | Leu |
| | | U-3 | | | | | U-4 | | |
| GGT | AAA | CTG | TCC | CAG | GAA | CTG | CAT | AAG | CTG |
| GGG | AAG | TTG | AGT | CAG | GAA | TTA | CAT | AAG | CTG |
| | | U-31 | | | | | U-41 | | |

In addition, the corresponding minus chains L3 and L4 are modified to L31 and L41.

The definitive sequences thus designed are shown in Fig. 1 and Fig. 2. Each of these sequences contains the structural gene coding for the salmon calcitonin consisting of 32 amino acids $Cys^1$ to $Pro^{32}$ or for the salmon calcitonin derivative consisting of 33 amino acids $Cys^1$ to $Gly^{33}$, the translation initiation codon ATG/TAC added at the 5'-end of the structural gene, and two translation termination codons TAA/ATT and TAG/ATC added at the 3'-end. To facilitate the integration of these DNA fragments into the plasmids, the HpaI-end or the sequence that provides the HpaI-end by treatment with the restriction enzyme HpaI is added on the 5'-side of the translation initiation codon, and the ClaI-end is added on the 3'-side of the translation termination codon. Of these structural genes, the salmon calcitonin structural gene is referred to as CT1 and the salmon calcitonin derivative structural gene is referred to as CT2.

The DNA sequence of the synthesis structural gene for the salmon calcitonin thus designed is as follows:

Code Chain: T G C T C C A A T C T C T C T A C T -
Anticode Chain: A C G A G G T T A G A G A G A T G A -
T G C G T T C T G G G G A A G T T G A G T -
A C G C A A G A C C C C T T C A A C T C A -
C A G G A A T T A C A T A A G C T G C A A -
G T C C T T A A T G T A T T C G A C G T T -
A C T T A C C C G C G T A C C A A C A C T -
T G A A T G G G C G C A T G G T T G T G A -
G G T T C T G G T A C A C C T
C C A A G A C C A T G T G G A

The DNA sequence of the salmon calcitonin derivative synthesis structural gene as follows:

Code Chain: T G C T C C A A T C T C T C T A C T -
Anticode Chain: A C G A G G T T A G A G A G A T G A -
T G C G T T C T G G G G A A G T T G A G T -
A C G C A A G A C C C C T T C A A C T C A -
C A G G A A T T A C A T A A G C T G C A A -
G T C C T T A A T G T A T T C G A C G T T -

A C T T A C C C G C G T A C C A A C A C T -
T G A A T G G G C G C A T G G T T G T G A -

For the synthesis of the thus designed oligonucleotides of the present invention, U1 or U11, U2, U31, U41, U5, U6, and U7 or U7' are synthesized for the code chain, and L1 or L11, L2, L3, L31, L41, L5, L6, L7 and L8 or L8' are synthesized for the anticode chain. The oligonucleotides thus synthesized are combined to synthesize chemically a DNA fragment containing the intended structural gene. Of the oligonucleotides designated above, those designated as U1 and L1 form the HpaI-end and those designated as U11 and L11 form the recognition sequence for the restriction enzyme HpaI. U7 and L8 are the oligonucleotides for the synthesis of a DNA fragment containing the salmon calcitonin derivative structural gene (CT2) while U7' and U8' are the oligonucleotides for the synthesis of a DNA fragment containing the salmon calcitonin structural gene (CT1).

In the synthesis of the intended DNA fragment from such oligonucleotides it is preferable to first construct a plurality of subblocks from the oligonucleotides and then combine these subblocks to give the intended DNA fragment. In the process of the present invention, various subblocks were synthesized as shown in Fig. 3, wherein the crossbar represents an oligonucleotide and the asterisk at one end of the crossbar represents a phosphorylated 5'-end. As an example of the method for combining these subblocks, the following scheme may be employed:

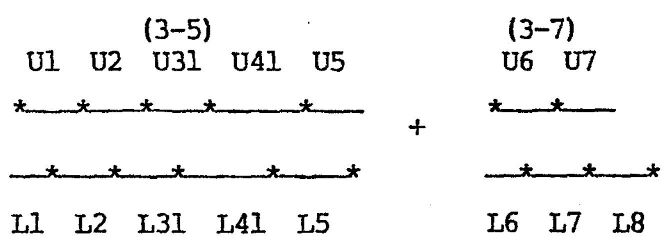


(I)

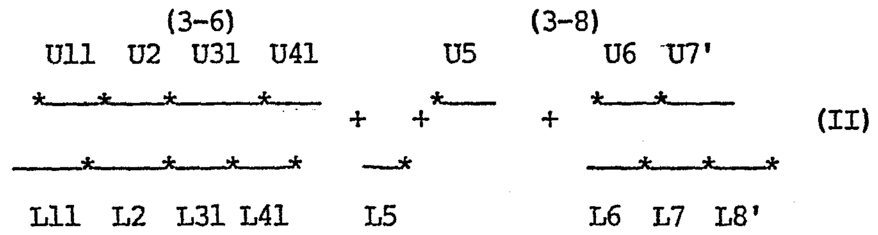


Scheme (I) gives a DNA fragment containing the salmon calcitonin derivative structural gene with the 5'-end serving as an HpaI-end, and scheme (II) gives a DNA fragment containing the salmon calcitonin structural gene with the restriction enzyme HpaI recognition sequence at the 5'-end.

The phosphotriester method which involves constructing the designed oligonucleotide from small units and the method involving the use of enzyme to combine oligonucleotides are well known (see H. Hsivng et al, <u>Nucleic Acids Research</u>, <u>6</u> 1371, 1979, and K. Agarwal et al, <u>Nature</u> <u>227</u> 27, 1970). It is preferable to use the solid-phase phosphotriester method for synthesizing oligonucleotides.

As an example of the synthesis, an L-31 oligonucleotide synthesis is given below.

The hydroxyl group at the 5'-position of benzoyldeoxycytidine (deoxycytidine is hereinafter simply referred to as cytidine and the same is true of deoxyguanosine and deoxyadenosine) fixed on a resin carrier through the hydroxyl group at the 3'-position is allowed to condense with a hydroxyl group at the 3'-position of guanosine of cytidine-guanosine(CG) dimer unit wherein bases, an internucleotide phosphate group, and a hydroxyl group at the 5'-position are protected, using a phosphate group activating agent.

Then the protecting group for hydroryl group at 5' of the newly added cytidine is eliminated, and condensation with the adenosine-adenosine (AA) dimer unit is effected. The oligonucleotide L-31 containing the protecting group is synthesized by successively condensing the subsequent dimer units AG, CC, CC, TT, and AC in such a manner.

The oligonucleotide L-31 is obtained by completely eliminating the protecting group at the 3'-position of the resin-linked benzoylcytidine, base protecting groups, internucleotide phosphate protecting group, and protecting group at the 5'-position of adenosine at the end of the oligonucleotide chain.

The use of enzymatic reaction is preferred for the synthesis of the structural gene by combinng oligonucleotides. A phosphate group is added to the oligonucleotide using polynucleotide kinase and adenosine triphosphate (ATP). A subblock forming a portion of the gene can be synthesized by first heating and then cooling a plurality of the oligonucleotides to cohere physically and then condensing mutually the phosphoryl groups at the 5'-ends of the oligonucleotides with the hydroxyl groups at the 3'-ends through the action of T4 polynucleotide ligase. The structural gene may be constructed by condensing subblocks mutually in a similar manner. Figure 4 is a radioautogram showing the construction of a CT2-containing DNA from the subblocks 3-5 and 3-7.

The DNA fragment containing the structural gene for salmon calcitonin or its derivative thus synthesized may be inserted into an appropriate plasmid vector by well-known methods. Plasmid pSCT1 is obtained by inserting the DNA fragment containing the salmon calcitonin structural gene (CT1) into plasmid pHT2, and plasmid pSCT2 is obtained by inserting the DNA fragment containing the salmon calcitonin derivative structural gene (CT2) into the same plasmid (Fig. 5).

The transformant obtained by transforming the strain *Escherichia coli* RR1 with the plasmid pSCT2 was designated as *Escherichia coli* RR1/pSCT2 and was deposited on August 17, 1984 at the Fermentation Research Institute, Agency of Industrial Science and Technology, Ministry of International Trade and Industry, at 1-3, Higashi 1-chome, Yatabe-cho, Tsukuba-gun, Ibaraki-ken, Japan (hereinafter abbreviated as FRI) as FERM P-7775. This transformant was placed under international deposition as FERM BP-866 on August 14, 1985 under the Budapest Treaty on the International Recognition of the Deposit of Microorganisms for the Purpose of Patent Procedure (hereinafter referred to as international deposition). Another transformant obtained by transforming Strain *Escherichia coli* RR1 with the plasmid pSCT1 was designated as *Escherichia coli* RR1/pSCT1 and was deposited with the FRI as FERM P-7774 on August 17, 1984.

(2) DNA Fragment Coding for Fused Protein

In order to produce a low molecular peptide such as calcitonin, it is generally considered advisable to first recover the material in the form of a stable high molecular protein fused with another protein and then cut it *in vitro* to give the desired low molecular peptide. In view of ease of purification, the use of metapyrocatechase or a portion thereof as a protein consisting of the fused protein in the production of the salmon calcitonin derivative of the present invention is preferred. It is also preferable to cause methionine to intervene at the cutting site, that is, between the first amino acid of the calcitonin derivative and the C-end amino acid of the metapyrocatechase protein, to obtain the intended calcitonin derivative after cutting the recovered fused protein.

In accordance with the present invention, therefore, a DNA fragment consisting of a gene coding for the salmon calcitonin derivative and a metapyrocatechase gene or portion thereof positioned upstream of the salmon calcitonin derivative gene with the genes being interrupted with the methionine codon ATG, may be used. It is preferred that this metapyrocatechase gene or the portion thereof consist of the SD sequence for the metapyrocatechase gene and the metapyrocatechase structural gene or the portion thereof to achieve a high translation efficiency.

The size of the metapyrocatechase structural gene segment is widely variable. For example, a structural gene coding for about 35 to 200 amino acids of the metapyrocatechase may be used. Where structural genes coding respectively for 37, 98, 142, and 197 amino acids of the metapyrocatechase are used in the process of the present invention, experssion of the fused protein containing the peptide of the salmon calcitonin derivative of the present invention was verified.

(3) Expression Plasmid

The expression plasmids of the present invention are *E. coli* plasmids containing an *E. coli* promoter or phage promoter, the fused protein gene, and a terminator, in that order. For example lacUV5, $P_L$, or tac promoter may be used as the promoter and $\lambda t_{L1}$ or trp attenuator may be used as the terminator.

Specifically, some examples of expression plasmids are cited in the following tabulation.

| Plasmid | Promoter | Terminator | No. of Amino Acids of Fused Protein |
|---|---|---|---|
| pLMC2 | lacUV5 | $\lambda t_{L1}$ | 231 |
| pPMC2 | $P_L$ | $\lambda t_{L1}$ | 231 |
| pTMC2 | tac | trp a | 176 |
| pTMC22 | tac | trp a | 71 |
| pTMC32 | tac | trp a | 132 |

(4) Transformant *E. coli*

As the host *E. coli* capable of expression of fused protein by afore-mentioned expression plasmid, strains such as RB791, HB101, JM103, C600, RR1, SM32 and W3110 may be used.

(5) Construction of Various Plasmids and Verification of Expression Starting Plasmid

In the construction of the aforementioned plasmids in accordance with the present invention, the DNA fragment containing the structural genes is cut off from the plasmid pSCT2 containing the calcitonin derivative structural gene, using appropriate restriction enzymes, and the fragment is inserted into an appropriate expression plasmid.

Figures 6 and 7 illustrate the construction of the expression plasmids of the present invention. In these figures, pSCT2 is a starting plasmid containing the calcitonin derivative structural gene and the plasmids in the boxes are expression plasmids of the present invention.

Plasmid pSLMK1 is a plasmid containing a promoter lacUV5 and the metapyrocatechase gene (C230), and the strain of E. coli containing this plasmid, i.e., Escherichia coli HB101/pSLMK1, W3110/pSLMK1, RR1/pSLMK1, and RB791/pSLMK1 were respectively deposited with FRI on May 11, 1984 as FERM P-7616, FERM P-7617, FERM P-7618 and FERM P-7619.

Plasmid pHT3 is a plasmid containing promoter $P_L$ and the metapyrocatechase gene (C230), and the strains of E. coli containing this plasmid, i.e., Escherichia coli HB101/pHT3, C600/pHT3, W3110/pHT3 were deposited with FRI on August 17, 1984 as FERM P-7776, FERM P-7777 and FERM P-7778, respectively.

Plasmid pST21 is a plasmid containing a tryptophan promoter/operator (trp P/0) and a reader region.

Plasmid pTCCM1 is a plasmid containing a tac promoter and a metapyrocatechase gene (C230), and the strains of E. coli, i.e., Escherichia coli

RB791/pTCCM1 and JM103/pTCCM1 containing this plasmid were deposited with FRI on August 17, 1984 as FERM P-7780 and FERM P-7781, respectively. The latter strain was placed under international deposition on August 8, 1985 as FERM BP-862.

Plasmid pUC9 is a well-known plasmid, J. Vieira and J. Messing, Gene Vol. 19, p.259-p.258 (1982).

Expression Plasmid pLMC2

In accordance with the method described in Example 4 (Fig. 6 and Fig. 8(A)), the expression plasmid pLMC2 is constructed from the plasmids pSLMK1 and pSCT2. Plasmid pSCT2 is constructed using the method described in Example 1. Plasmid pLMC2 contains the salmon calcitonin derivative gene downstream of, and in frame with a lacUV5 promoter and subsequent gene for a portion of metapyrocatechase (C230), and the $\lambda t_{L1}$ terminater is positioned downstream of the salmon calcitonin derivative gene. The connecting site of the metapyrocatechase structural gene and the salmon calcitonin derivative structural gene has the nucleotide sequence as shown in Fig. 8(B). The structural gene region in this plasmid codes for a fused protein consisting of 197 amino acids of the metapyrocatechase, methionine, and 33 amino acids of the salmon calcitonin derivative (a total of 231 amino acids).

*Escherichia coli* JM103/pLMC2 containing this plasmid was deposited with FRI on May 10, 1985 as FERM P-8220 and was placed under international deposition on August 18, 1985 as FERM P-867.

*Escherichia coli* RR1 was transformed with the plasmid, cultured in an LB medium with and without isopropyl-β-D-thiogalactopyranoside (hereinafter abbreviated as IPTG), the bacterial cells harvested, the protein extracted from the cells, and the extract subjected to SDS polyacrylamide gel electrophoresis. The electrophoretic analysis verified that about 25,000 daltons of the protein had been induced in the presence of IPTG. Furthermore, this protein was shown by the western blot technique to react with the antibody to the salmon calcitonin I and the 25,000 daltons of the expression product consequently identified as the fused protein of a portion of the metapyrocatechase and the salmon calcitonin derivative.

Expression Plasmid pPMC2

Expression plasmid pPMC2 is constructed from plasmids pLMC2 and pHT31 by the method described in detail in Example 5 (Figs. 6 and 9). Plasmid pHT31 is constructed from plasmid pHT3 as described in Example 2. This plasmid contains the salmon calcitonin derivative gene downstream of, and in frame with $P_L$ promoter and subsequent gene for a portion of metapyrocatechase (C230), and the $\lambda t_L$-terminater is present downstream of the salmon calcitonin derivative gene. The structural gene region in this plasmid codes for a fused protein consisting of 197 amino acids of the metapyrocatechase, methionine, and 33 amino acids of the salmon calcitonin derivative (a total of 231 amino acids).

Escherichia coli RR1/pPMC2 containing the plasmid was deposited with FRI on May 10, 1985 as FERM P-8222 and was placed under international deposition on August 14, 1985 as FERM BP-868.

The strain of E. coli transformed with the plasmid was verified as described hereinabove to produce about 25,000 daltons of the fused protein consisting a portion of the metapyrocatechase segment and the salmon calcitonin derivative.

Expression Plasmid pTMC2

Expression plasmid pTMC2 is constructed from plasmids pTCCMAX and pUCT22 as described in Example 10 (Figs. 7 and 12). Plasmid pTCCMAX is constructed from plasmid pTCCM1 as described in Example 9 (Figs. 7 and 12A). Plasmid pUCT22 is constructed from plasmids pSCT2, pUC9, pST21 and pBR322 using the methods described in Examples 6, 7 and 8 and Example 3 (Figs. 7, 10, 11 and 16). Plasmid pTMC2 contains the salmon calcitonin derivative structural gene downstream of, and in frame with the tac promoter and subsequent gene for a portion of metapyrocatechase, and trp attenuator is present downstream with the salmon calcitonin derivative structural gene. The base sequence at the connection site of the region coding for a portion of the metapyrocatechase and the region coding for the salmon calcitonin derivative is shown in Fig. 12B.

The structural gene region in this plasmid codes for the fused protein consisting of 142 amino acids of the metapyrocatechase, methionine and 33 amino acids of the salmon calcitonin (a total of 176 amino acids).

Escherichia coli JM103/pTMC2 containing the plasmid was deposited with FRI on May 10, 1985 as FERM P-8223 and was placed under international deposition on August 14, 1985 as FERM BP-869.

Expression Plasmid pTMC22

Expression plasmid pTMC22 is constructed from plasmids pTMC12 and pTCCMAX by the method described in detail in Examples 11 and 12 (Figs. 7 and 13). The plasmid pTMC22 contains the salmon calcitonin derivative structural gene downstream of, and in frame with tac promoter and subsequent gene for a portion of metapyrocatechase (C230), and trp attenuator exists downstream of the salmon calcitonin derivative structural gene. The structural gene region in the plasmid codes for a fused protein consisting of 37 amino acids of the metapyrocatechase, methionine and 33 amino acids of the salmon calcitonin derivative (a total of 71 amino acids).

Expression Plasmid pTMC32

Expression plasmid pTMC32 is constructed from plasmids pTMC12 and pTMC22 by the method described in Example 13 (Figs. 7 & 14). The plasmid pTMC32 contains the salmon calcitonin derivative gene downstream of, and in frame with the lac promoter and subsequent gene for a portion of metapyrocatechase (C230), and trp attenuator exists downstream of the salmon calcitonin derivative gene.

The structural gene region in the plasmid codes for the fused protein consisting of 98 amino acids of the metapyrocatechase molecule, methionine and 33 amino acids of the salmon calcitonin derivative (a total of 132 amino acids).

*Escherichia coli* JM103/pTMC32 containing the plasmid was deposited with FRI as FERM P-8221.

Strain of *E. coli* RB791 transformed by any one of the three plasmids pTMC2, pTMC22, and pTMC32 was cultured overnight in an LB medium containing 50 µg/ml of ampicillin, 0.05 ml of the cultured broth was added to 5 ml of the same medium, and culturing was carried out at 37°C with shaking. Experiments were performed on each strain with and without induction by IPTG. Where the IPTG induction was conducted, IPTG was added to bring the final concentration to 1 mM. Culturing was carried out for 8 hours in all.

At the end of the culturing, the cells were harvested, the whole protein extracted, and the extract subjected to SDS polyacrylamide gel electrophoretic analysis. The results showed that the addition of IPTG remarkably induced the production of a protein having a molecular weight of about 19,000 in the strain containing pTMC2, and of a protein having a molecular weight of about 14,000 in the strain containing pTMC32.

2. Construction of Gene System for Eel Calcitonin Derivative

(1) Construction of Eel Calcitonin Derivative Structural Gene

As stated earlier, in the eel calcitonin derivative, an amino acid at 26, i.e., asparagine in the salmon calcitonin derivative, is replaced by aspartic acid, an amino acid at 27, i.e.,

threonine, is replaced by valine, and an amino acid at 29, i.e., serine, is replaced by alanine. A gene for the eel calcitonin derivative, wherein only three amino acids are different from corresponding amino acids in the salmon calcitonin as described above, is advantageously synthesized starting from the salmon calcitonin derivative gene already synthesized, according to a known mutagenesis such as site-directed mutagenesis using an oligonucleotide primer. The process of the present invention therefore comprises converting codons AAG, ACT and TCT respectively for asparagine at 26 position, threonine at 27 position, and serine at 29 position in the amino acid sequence of the salmon calcitonin derivative to codons GAC, GTT, and GCT respectively for aspartic acid, valine and alanine by subjecting the salmon calcitonin derivative gene to the site-directed mutagenesis to give the desired eel calcitonin derivative gene. Because of the mutual proximity of these codons in the base sequence, the mutation may be carried out using a single-primer oligonucleotide, having, for example, the following sequence:

5' TGG TCG TGG TTG CAG CCA TGC GC 3'
Ala Var Asp

For example, the mutation is carried out in the following manner: The plasmid pSCT2 is cut by restriction enzymes Hpa I and Sal I to give a 0.6 kbp fragment. On the other hand, the double-stranded DNA of the phage M 13mp9 is cut by Sma I and Sal I to give a 2.7 kbp fragment. These fragments are ligated using $T_4$DNA ligase and the ligation product is used to transform E. coli JM103. This transformant is cultured to obtain single-stranded recombinant phage DNA. The single-stranded DNA contains an anticoding chain (minus chain) of the salmon calcitonin derivative. This recombinant phage is designated as CTM31, which serves as a template for forming double-stranded DNA using the primer. After purification, the double-stranded DNA is used to transform E. coli JM103. The phage plaque is then screened using the following $^{32}P$-labeled synthetic oligonucleotide probe to obtain mutant phages:

5' TCGTGGTTGCAGCCA 3'

The nucleotide sequence of the phages is determined, and a phage containing the intended mutated gene (eel calcitonin derivative gene) is selected. This phage is designated as CTM41.

The resulting gene coding for the eel calcitonin derivative has the following nucleotide sequence:

Coding Chain: T G C T C C A A T C T C T C T A C T -
Anti-coding Chain: A C G A G G T T A G A G A G A T G A -
T G C G T T C T G G G G A A G T T G A G T -
A C G C A A G A C C C C T T C A A C T C A -
C A G G A A T T A C A T A A G C T G C A A -
G T C C T T A A T G T A T T C G A C G T T -
A C T T A C C C G C G T A C C G A C G T T -
T G A A T G G G C G C A T G G C T G C A A -
G G T G C T G G T A C A C C T G G T
C C A C G A C C A T G T G G A C C A

The nucleotide sequences of the salmon calcitonin derivative gene (a part of phage CTM 31), the eel calcitonin derivative gene (a part of phage CTM41), the primer oligonucleotide, and of the probe oligonucleotide are shown in Fig. 17 along with the amino acid sequences of the salmon calcitonin and eel calcitonin derivatives.

(2) DNA Fragment Coding for Fused Protein

As stated earlier, in order to produce low-molecular peptides such as calcitonin using E. coli, it is generally considered advisable to first recover the low-molecular peptide in the form of a stable high-molecular protein fused with another protein and then cut the fused protein to give the desired low-molecular peptide. In the process of the present invention for the production of the eel calcitonin derivative, metapyrocatechase or its portion is preferably used as a protein for formation of such a fused protein, to facilitate purification. In order to obtain the intended calcitonin derivative after recovering and cutting the fused protein, it is also preferable that methionine be present at the cutting site, i.e., between amino acid 1 of the calcitonin derivative and the C-terminal amino acid of the metapyrocatechase protein.

Therefore, in accordance with the present invention, for example, the DNA fragment for the fused protein preferably consists of calcitonin derivative gene and a metapyrocatechase gene or a part thereof positioned upstream of the calcitonin derivative gene with the genes being interrupted with the codon ATG for the methionine. It is preferred that this metapyrocatechase gene or a part thereof be composed of the SD sequence of the metapyrocatechase gene and the metapyrocatechase structural gene or a portion thereof to achieve a high translation efficiency. The size of the part of the metapyrocatechase structural gene is widely variable.

(3) Expression Plasmid

The expression plasmids of the invention are E. coli plasmids containing an E. coli promoter and a gene for the fused protein, in that order. For example, lacUV5, $P_L$ , and tac promoters may be used.

As a particular example of the expression plasmids, the expression plasmid pCTM4 obtained by inserting an eel calcitonin derivative gene in the phage CTM41 into the plasmid pTCCM1 for expression may be cited.

The plasmid pTCCM1 contains a tac promoter and a metapyrocatechase gene (C230) positioned downstream of that promoter. Each strain of E. coli containing the plasmid, i.e., Escherichia coli RB791/pTCCM1 and JM103/pTCCM1 respectively were deposited with FRI on August 17, 1984 as FERM P-7780 and FERMP-7781 respectively.

In the construction of the plasmid pCTM4, as shown in Fig. 8(A), a double-stranded form of phage CTM41 containing the eel calcitonin derivative gene (shown as CT in the figure) is digested with EcoRI to cut the phage at the EcoRI site located downstream and in the vicinity of methionine at 0 position of the eel calcitonin gene, and is filled-in to form blunt ends. The SalI site downstream of the eel calcitonin gene is then cut by SalI and a small 590 bp fragment containing the eel calcitonin derivative gene is isolated.

On the other hand, plasmid pTCCM1 is digested with Ava I to cut an Ava I site positioned midway of the metapyrocatechase gene (shown as C230 in the figure) in the plasmid, filled-in to form blunt ends. The SalI site positioned downstream of the metapyrocatechase is then cut by SalI, and a large 2940 bp fragment, from which the downstream portion of the metapyrocatechase gene has been eliminated, is isolated.

These two fragments are ligated with T4 DNA ligase, and the ligation product is used to transform E. coli JM103, and clones containing a plasmid, into which the eel calcitonin derivative gene has been properly inserted, are selected by cloning hybridization, nucleotide sequence determination, etc. One such plasmid is designated as pCTM4. The nucleotide sequence in the region where the upstream site of the eel calcitonin derivative gene (derived from CTM41) connects to the downstream site of the metapyrocatechase gene (derived from pTCCM1) in this plasmid is shown in Fig. 18(B).

The plasmid pCTM4 contains a gene coding for a fused protein (total of 174 amino acids) consisting of amino acids of the metapyrocatechase and 33 amino acids of the eel calcitonin derivative positioned downstream of the metapyrocatechase amino acids, under the control of a tac promoter. Escherichia coli JM103/pCTM4 containing this plasmid was deposited with FRI on May 20, 1985 as FERM P-8239) and was placed under international deposition on August 14, 1985 as FERM BP-870.

3. Construction of Gene System for Other Calcitonin Derivatives

The construction of a gene system for the salmon calcitonin derivative, and a gene system for the eel calcitonin derivative starting from the salmon calcitonin derivative structural gene has been described in the preceding paragraphs. Gene systems for a variety of calcitonin derivatives falling within the prescribed scope and purview of the present invention may be similarly constructed by assigning the particular amino acids defined in the general formula (I) to X, Y and Z in the amino acid sequence shown in the general formula. That is, while the structural gene for the eel calcitonin derivative of the present invention was constructed by subjecting the corresponding sites (those coding for amino acids at 26, 27 and 29) of the salmon calcitonin derivative structural gene to the site-directed mutagenesis using a specific primer oligonucleotide, desired calcitonin derivative structural genes may be constructed by modifying the corresponding nucleotide sequence using primer oligonucleotides containing an appropriate nucleotide sequence.

Expression plasmids may be constructed by manipulating the structural genes thus constructed in the same way as the salmon calcitonin derivative genes or eel calcitonin derivative genes, and desired calcitonin derivatives defined by the general formula (1) may be produced by the method similar to those described later in detail for the salmon calcitonin derivative and the eel calcitonin derivative using the plasmids thus constructed.

4. Production and Isolation of Fused Protein and Preparation of Fish Calcitonin Derivative

Fused protein-producing strains of E.coli may be obtained by introducing one of the variety of expression plasmids into E. coli. Strains such as RB791, JM103, W3110, RR1, and SM32 may be used as hosts, and, for example, strains such as W3110 and RB791 are preferred as hosts when the plasmid pLMC2 is used.

Culturing is carried out in an LB medium under aerobic conditions using the conventional method. The addition of about 50 μg/ml. of ampicillin to the medium is preferred in order to sustain the host containing the intended plasmid. When the cell density has reached the desired level, for instance, optical densities 0.3 to 0.6 at 550 nm, IPTG is added to the extent that the final concentration thereof reaches 1 mM and culturing is continued for an additional 2 to 8 hours.

At the end of the culturing period the cells are removed using a conventional method such as centrifugation or filtration, and the cells are washed as desired with an appropriate buffer such as 50 mM potassium phosphate-10mM EDTA buffer, pH 7.5. The cells are then disintegrated using a conventional method such as lysozyme treatment or ultrasonication, and the precipitated fractions are recovered by centrifugation. As desired, the precipitates are resuspended with a 50 mM potassium phosphate buffer, pH 7.5, the suspension recentrifuged, and the precipitates recovered.

Most of the intended fused protein is recovered in the precipitated fraction and the contaminant proteins dissolved in the supernatant fractions are removed by such treatment. The simplicity of the operation by which the intended fused protein can be separated from the contaminant proteins is one of the prominent features of the present invention, enabling the fish calcitonin derivative to be expressed as a fused protein product with metapyrocatechase.

In order to allow the resulting insoluble fraction to be further purified, the fraction is dissolved in an aqueous guanidine hydrochloride solution, the fused protein allowed to precipitate by removing guanadine hydrochloride on dialysis of the solution, and the precipitate is recovered by centrifugation. Further purification may be achieved as desired by redissolving the precipitate in an aqueous guanadine hydrochloride solution and subjecting the suspension to Sephadex G75 column chromatography.

The fused protein thus recovered is cut by the CNBr using the conventional method and the intended fish calcitonin derivative is liberated.

Finally, the crude fish calcitonin derivative is purified using the conventional method. For example, C18, $C_4$RP, and TSK G2000 SW column chromatographies are suitable for use in this purification step.

C. Properties of Fish Calcitonin

(1) Analysis of Purified Salmon Calcitonin Derivative

The result of amino acid analysis is given in the following table.

| Amino Acid | Measured Value | Theoretical Value |
|---|---|---|
| Asp | 1.96 | 2 |
| Thr | 4.21 | 5 |
| Ser | 3.43 | 4 |
| Glu | 3.04 | 3 |
| Gly | 3.98 | 4 |
| Ala | 0.12 | |
| Val | 1.08 | 1 |
| 1/2 Cys | 1.20 | 2 |
| Met | 0.03 | |
| Ile | 0.06 | |
| Leu | 5.05 | 5 |
| Tyr | 1.02 | 1 |
| Phe | 0.03 | |
| Lys | 1.97 | 2 |
| His | 1.06 | 1 |
| Arg | 1.03 | 1 |
| Pro | 1.70 | 2 |
| Trp | - | |
| Total | | 33 |

The theoretical value of Gly hereinabove is based on the assumption that the Gly is present at the C-end. The amino acid composition of the peptide resulting from the present invention was found to be in agreement with the value calculated for the intended salmon calcitonin derivative.

Amino acid sequencing using a protein sequencer confirmed that the sequence of all amino acids is identical with that of the intended product.

(2) Analysis of Purified Eel Calcitonin Derivative

The result of amino acid analysis is given in the following table.

| Amino Acid | Measured Value | Theoretical Value |
|---|---|---|
| Asp | 2.2 | 2 |
| Thr | 3.6 | 4 |
| Ser | 2.6 | 3 |
| Glu | 3.4 | 3 |
| Gly | 4.1 | 4 |
| Ala | 1.3 | 1 |
| Val | 2.1 | 2 |
| 1/2 Cys | 1.3 | 2 |
| Met | | |
| Ile | | |
| Leu | 5.1 | 5 |
| Tyr | 1.0 | 1 |
| Phe | | |
| Lys | 2.1 | 2 |
| His | 1.0 | 1 |
| Arg | 1.2 | 1 |
| Pro | 2.0 | 2 |
| Trp | | |
| Total | | 33 |

The theoretical value of Gly hereinabove is based on the assumption that the Gly is present at the C-end. The amino acid composition of the peptide resulting from the present invention was found to be in agreement with that of the eel calcitonin described in available literature, having an additional glycine residue attached to the molecule.

Amino acid sequencing using a protein sequencer confirmed that the amino acid sequence is identical to that of the expected eel calcitonin derivative.

(3) Physiological Activity of Fish Calcitonin Derivative

The fish calcitonin derivative per se of the present invention possesses the physiological activities found in calcitonin. For example, the activity level of the salmon calcitonin derivative in mammals is about one seventh that of the salmon calcitonin, which is equal or greater than that of the human calcitonin. The fish calcitonin derivative of the present invention is therefore useful as a pharmaceutical. The physiological activity of this derivative was proved as follows:

One hundred and twenty 3-week-old male Crj: Wistar rats (Japan Charles River, Ltd.) were purchased, and after one week of conditioning, 110 healthy 4-week-old animals were used.

The animals were kept in a room automatically conditioned to an environment of 22°C ± 2°C of temperature, 55% ± 10% of humidity, 13 times/hour of air exchange, and 12-hour illumination (7 A.M. - 7 P.M.). The animals were housed in metal net cages (250 mm x 350 mm, Japan Cage, Ltd.) in groups each consisting of 5 animals and were allowed free access to the same food (MF: Oriental Yeast Industry, Ltd.) and to city water.

The salmon calcitonin derivative prepared by the process of the present invention (a white lyophilized preparation, hereinafter referred to as SGE-1) and the commercially available salmon calcitonin I (a white lyophilized preparation, hereinafter referred to as CT) were used as subject compounds.

These subject substances were dissolved in the following solvents and 10,000 ng/m of subject solutions were prepared:

| | |
|---|---|
| Sodium citrate | 1.3 mM |
| Citric Acid | 22 mM |
| Sodium Chloride | 120 mM |
| Gelatin | 0.16% |
| pH | 6.0 |

Seven dose levels were set for SGE-1 with maximum dose levels of 400 ng/kg and subsequently 283 ng/kg, 200 ng/kg, 141 ng/kg, 100 ng/kg, 71 ng/kg and 50 ng/kg at a common ratio of 2, and four levels were set for CT with a maximum level of 40 ng/kg and subsequently 20 ng/kg, 10 ng/kg and 5 ng/kg at a common ratio of 2. The subject solutions were diluted with the solvent so that the amount administered would be exactly 1 ml per 100g body weight for each dose level.

The animals were fasted after 6 P.M. on the day before drug administration and were weighed after the 110 animals had been randomly divided into 11 groups each comprising 10 animals. One milliliter per 100g body weight of the subject solution was administered caudointravenously to each animal on the basis of individual body weights. After exactly one hour, blood samples were taken from an abdominal artery of the individual animals under ether anesthesia. The samples were allowed to stand for about one hour at room temperature and the blood serum was obtained after centrifugation for 10 minutes at 3000 rpm. The blood serum samples were used for the measurement of calcium concentrations by the O-Cresolphthaien Conplexone method. Clinical examinational autoanalyzer JCA-VX1000 (Japan Electron Co.) was used for the measurement of calcium levels and double-measured mean values were used as measured values of the calcium concentrations.

The data from these measurements was first examined for isodispertivity in each group of animals using the Bartlet method. Because of the absence of negative isodispersivity, relative activities of SGE-1 to CT were evaluated by 2 x 3-point parallel-line tests. The results are given in the following table.

| Subject Substance | CT | | | | SGE-1 | | | | | | |
|---|---|---|---|---|---|---|---|---|---|---|---|
| Dose | 5 | 10 | 20 | 40 | 50 | 71 | 100 | 141 | 200 | 283 | 400 |
| No. | | | | | | | | | | | |
| 1 | 9.0 | 8.4 | 7.9 | 7.2 | 8.9 | 9.3 | 8.6 | 8.2 | 7.2 | 7.8 | 6.8 |
| 2 | 8.6 | 8.4 | 7.6 | 7.0 | 9.3 | 9.0 | 8.1 | 7.2 | 7.3 | 6.3 | 6.4 |
| 3 | 8.8 | 8.3 | 7.0 | 6.9 | died | 7.9 | 8.0 | 7.3 | 7.5 | 6.8 | 6.4 |
| 4 | 8.5 | 7.9 | 7.7 | 6.6 | 8.9 | 7.9 | 7.3 | 7.6 | 7.6 | 7.0 | 6.9 |
| 5 | 8.5 | 8.7 | 7.7 | 6.8 | 9.0 | 8.1 | 8.6 | 7.6 | 7.8 | 6.9 | 6.3 |
| 6 | 8.5 | 7.6 | 7.4 | 6.4 | 8.3 | 8.4 | 8.1 | 8.0 | 7.1 | 6.6 | 6.1 |
| 7 | 8.6 | 8.7 | 7.3 | 7.0 | 8.3 | 8.2 | 8.0 | 7.0 | 7.0 | 6.8 | 7.1 |
| 8 | 8.5 | 7.9 | 7.0 | 6.8 | 8.4 | 7.9 | 7.3 | 7.5 | 6.6 | 6.6 | 6.2 |
| 9 | 8.5 | 8.4 | 7.4 | 7.1 | 8.8 | 8.0 | 7.9 | 7.7 | 6.9 | 7.2 | 6.7 |
| 10 | 9.0 | 8.6 | 7.6 | 6.5 | 8.6 | 8.7 | 8.5 | 6.8 | 7.3 | 6.9 | 7.7 |
| Mean | 8.65 | 8.29 | 7.62 | 6.83 | 8.72 | 8.34 | 8.04 | 7.49 | 7.23 | 6.89 | 6.66 |
| Standard Variation | 0.21 | 0.37 | 0.20 | 0.26 | 0.35 | 0.50 | 0.47 | 0.43 | 0.35 | 0.40 | 0.49 |

(1) Dose shown in g/kg.

(2) Measured value shown in mg/dl.

Of the CT-administered groups, the groups at 10 ∿ 40 ng/kg dose levels showed a linear dose correlation and a similar dose correlation was observed for the SGE-1 groups at dose levels of 50 ∿ 283 ng/kg. Since the same number of samples are required for each dose level in parallel-line tests, the group at the 50 ng/kg level of SGE-1 was not available for the testing. Therefore, 2 x 3-point parallel-line tests were performed using the groups at 10, 20, and 40 ng/kg dose levels of CT and the groups at 71, 141, and 283 ng/kg levels of SGE-1 for the sake of equality in the common ratio. As the result, SGE-1 was shown to have an activity equivalent to about 0.14 times as much as CT on a per unit weight basis, with under its 95% confidence limit, it being from about 0.11 to 0.17 times as much as CT.

Since salmon calcitonin is said to be active in humans about 20 to 30 times as much as human calcitonin, the above value strongly suggests that the salmon calcitonin derivative of the present invention exhibits about 3 to 4 times as much activity in humans as does human calcitonin.

(4) Utility of Fish Calcitonin Derivative as Precursor for Natural Fish Calcitonin

The fish calcitonin derivative of the present invention may be converted to fish calcitonin, in which amino acid 32, i.e. proline, is amidized, by converting amino acid 33, i.e. glycine, to amide using a well-known method, e.g., carboxylpeptidase, K. Bleddan, F. Widmer & J.T.Johansen, Carlsberg Res. Commun., 45,237; 45,361 (1980). The fish calcitonin derivative of the present invention is therefore useful as an intermediate for preparing high-activity natural fish calcitonin.

The following examples are provided solely for further illustration:

Example 1 Synthesis of Genes for Salmon Calcitonin and Its Derivative

Synthesis of Oligonucleotide

Chemical synthesis of 23 different oligonucleotides shown in Figs. 1 to 3 was carried out using the phosphotriester solid-phase method. For example, in order to synthesize completely protected L-31 oligonucleotide, 40 mg (2.2 μmol) of cytosinenucloside was allowed to react sequentially with 15 mg each of dimer units CG and AA, and 10 mg each of dimer units AG, CC, CC, TT and AT.

A single condensation operation embraces the following steps: Reaction was carried out in 350 μl of anhydrous pyridine at room temperature for one hour using 20 mg of 2, 4, 16-trimethylbenzenesulfonyl-3-nitrotriazolide (MSNT). After reaction, the solids were washed with pyridine and subjected to capping-reaction in 10% anhydrous acetate pyridine solution for 3 minutes at room temperature using dimethylaminopyridine as a catalyst. After washing respectively with pyridine and dichlormethane solutions, the dimethoxytriethyl (DMTr) group which is a protecting group at the 5'-end was eliminated by washing the solid material with 10 ml of a 3% TCA dichlormethane solution. The solid material was then washed with dichlormethane and tetrahydrofuran (THF), and the moisture in the vessel was completely eliminated by azeotropic dehydration with pyridine, making it ready for the next condensation reaction. The mean condensation yield calculated on the basis of the DMTr group elimination rate was 91%.

To oligonucleotide L-31 containing the protecting groups synthesized from 40 mg of nucleoside resin by sequential condensation reaction were added 1.5 ml of 0.5M pyridinealdoxime and a 50% aqueous dioxane solution of tetramethylguanidine (TMG) and the mixture was subjected to reaction for 2 days at 30°C. After recovery of the solvent by concentration, the remaining product was reacted in a sealed tube with 2 ml of an aqueous pyridine solution containing 22% ammonia for 5 hours at 55°C.

The nucleotide was disconnected from the resin and protecting groups of the internucleotide-bonding phosphate groups and protecting groups of the nucleic acid bases were eliminated by these reactions.

After removal of the resin by filtration and of ammonia by concentration under reduced pressure, the resulting oligonucleotide containing a protecting group at the 5'-end was subjected to disposable column C-18 Seppak (Waters). After passing 20 ml of aqueous acetonitryle solution having a 15% concentration through the column, the intended product was eluted from the column on additoin of 2 ml of a 30% aqueous acetonitryle solution.

After addition of the same volume of acetic acid to the eluate, reaction was carried out for half an hour at room temperature to eliminate the DMTr group. After the acetic acid was eliminated by ether extraction and azeotropic dehydration, the solvent was replaced by ethanol, and the oligonucleotide was precipitated by centrifugation for 10 minutes at 3,000 rpm. After removal of the supernatant, the remaining ethanol was removed under reduced pressure and the precipitate was dissolved in 100 µl of distilled water.

Purification by HPLC was carried out using a µ Bondapak C-18 (Waters) column and a 0.1 M triethylamineacetate-acetonitryle solvent system. Starting with 10% the concentration of acetonitryle was escalated at a 0.6%/minute gradient for 60 minutes. Elution was carried out at a flow rate of 1 ml per minute, fractions with expected peaks collected using a fraction collector (Gilson), and the collected fractions were lyophilized.

The same recovering procedures were repeated for three portions to yield 21 OD (measured at 260 mµ) of a purified L-31 oligonucleotide.

Other oligonucleotides were synthesized in the same manner. The purity of the synthesized fragments was verified by the single-dimensional homochromatographic method. The $R_f$ values of 21 oligonucleotides representing their physical property determined by single-dimensional homochromatography are shown below.

| | | Sequence | Rf Value |
|---|---|---|---|
| U 1 | 16mer | A A C A T G T G C T C C A A T C | 0.14 |
| U 11 | 19mer | G T T A A C A T G T G C T C C A A T | 0.09 |
| U 2 | 17mer | T C T C T A C T T G C G T T C T G | 0.17 |
| U 3 | 15mer | G G T A A A C T G T C C C A G | 0.20 |
| U 31 | 15mer | G G G A A G T T G A G T C A G | 0.19 |
| U 4 | 15mer | G A A C T G C A T A A G C T G | 0.21 |
| U 41 | 15mer | G A A T T A C A T A A G C T G | 0.17 |
| U 5 | 15mer | C A A A C T T A C C C G C G T | 0.15 |
| U 6 | 17mer | A C C A A C A C T G G T T C T G G | 0.10 |
| U 7' | 18mer | T A C A C C T G G T T A A T A G A T | 0.10 |
| U 7 | 15mer | T A C A C C T T A A T A G A T | 0.17 |
| L 1 | 8mer | C A C A T G T T | 0.26 |
| L 11 | 15mer | C A C A T G T T A A C T G C A | 0.14 |
| L 2 | 17mer | A A G T A G A G A G A T T G G A G | 0.10 |
| L 3 | 15mer | G T T T A C C C A G A A C G C | 0.11 |
| L 31 | 15mer | A C T T C C C C A G A A C G C | 0.13 |
| L 4 | 15mer | G C A G T T C C T G G G A C A | 0.13 |
| L 41 | 15mer | G T A A T T C C T G A C T C A | 0.15 |
| L 5 | 16mer | T A A G T T T G C A G C T T A T | 0.14 |
| L 6 | 17mer | C A G T G T T G G T A C G C G G G | 0.08 |
| L 7 | 14mer | A G G T G T A C C A G A A C | 0.17 |
| L 8 | 13mer | C G A T C T A T T A A C C | 0.21 |
| L 8' | 10mer | C G A T C T A T T A | 0.25 |

The purity and nucleic acid sequence was verified by the two-dimensional homochromatographic method (e.g., R. Bambara et al, NAR 1 311, 1974) and the Maxam and Gilbert method (e.g., A. Maxam et al, Methods in Enzymology, 65 499, 1980) for half of the oligonucleotides.

Construction of Subblocks with Oligonucleotides

Some of the subblocks constructed by enzymatic reaction are shown in Fig. 3. For example, in order to construct the subblock 3-5, 100 pM each of 10 oligonucleotides constituting the subblock were diluted respectively with distilled water to 9 μl, and each of the diluted oligonucleotide was added with 10 μ Ci of $\gamma$-$^{32}P$ ATP(31000 Ci/mmol), adjusted to 50 mM Tris-hydrochloride, pH 9.6, 10 mM $MgCl_2$ , 2 mM spermine, 100 mM KCl, 10 mM DTT, and 4 units of polynucleotide kinase was added to bring the total volume to 15 μl. The 5'-end was labeled with $^{32}P$ by reaction at 37°C for 30 minutes. To phosphorylate the 5'-end of all oligonucleotides, 1 nM of ATP and one unit of polynucleotide kinase were added and reaction was carried out for 60 minutes at 37°C.

After the reaction, 10 oligonucleotides were mixed and the mixture was purified by the aforesaid method using a disposable column C-18 Seppak (Waters). The eluted DNA solution was concentrated and the concentrate was treated with ethanol to allow the DNA to precipitate. After removal of the supernatant, the remaining ethanol was eliminated under reduced pressure, the dried matter was dissolved in 40 μl of a ligase buffer (25 mM N-2-hydroxyethylpyperazine-N'-2-ethansulfonic acid (HEPES) pH 7.8, 7.5 mM $MgCl_2$). The solution was transferred into a 1.5 ml Eppendorf tube and after heating for 10 minutes at 65°C and then for 30 minutes at 42°C, the 10 fragments were joined at 0°C. The ligase solution containing the DNA's was adjusted to 0.2 mM of ATP and 6 mM of DTT, and 8 units of T4 ligase was added to bring the total volume to 50 µl, and reaction was carried out for 16 hours at 12°C.

A portion of the reaction mixture was withdrawn and was subjected to 12% polyacrylamide gel electrophoresis for evaluation. After the electrophoresis using two types of gel, one with 7 M urea and the other without, autoradiography was carried out and the composition of the reaction product was examined using a densitometer (Helena Laboratories). The results showed that DNA's corresponding to 78 bases and 93 bases had formed at the respective rates of 15% and 27%. the other blocks were synthesized in the same manner (Fig. 3).

Yields from the synthesis were as follows:

| Subblock | Yield (%) |
|---|---|
| 3-1 | 0.3 |
| 3-2 | 2 |
| 3-3 | 85 |
| 3-4 | 65 |
| 3-5 | 23 |
| 3-6 | 31 |
| 3-7 | 50 |
| 3-8 | 48 |

As can be seen from the above, although the yields of the subblocks 3-1 and 3-5 containing the nucleotide sequences which had not been modified according to the present invention were very low, those of the other subblocks according to the present invention were sufficiently high.

Construction of the Entire Genes from Subblocks

The entire sequence of nucleotides was constructed by linking the subblocks.

For example, in order to construct an entire gene by the method using the scheme (I) cited earlier, 4.5 µl each of the reaction mixtures resulting from ligase reaction of the subblocks 3-5 and 3-7 were mixed in a 1.5-ml Eppendorf tube, and after heating the mixtures for 10 minutes at 65°C and for 30 minutes at 42°C, the subblock DNA's were annealed at 0°C. To the DNA solution was added 30 nM of ATP and 500 nM of DTT and to this was added 12 units of T4 ligase to bring the total volume to 100 µl. The mixture was subjected to reaction for 16 hours at 12°C.

Examination for composition of the reaction product showed that DNA corresponding to pairing of 113 bases and 115 bases had formed at a rate of 23% of the total products.

The total volume of the reaction mixture was then separated by 12% polyacrylamide gel electrophoresis containing 7 M urea. After confirmation of the intended DNA by autoradiography, fragments were cut out from the corresponding area of the gel and transferred into a dialysis tube. The tube was filled with 89 mM Tris-borate, 2 mM EDTA buffer and sealed. DNA in the gel was eluted by subjecting the fragments to electrophoresis in the same buffer for 12 hours at a constant voltage of 50 V. The DNA was withdrawn from the dialysis tube and was lyophilized. The lyophilate was dissolved in 100 µl of distilled water and the crude DNA was purified using a disposable ion-exchange column Elutip-d (Schleicher & Schüell).

The subblocks 3-6 and 3-8 were combined via oligonucleotides L5 and U5 by the method using the scheme (II) cited earlier and DNA corresponding pairing of 113 bases and 115 bases was obtained.

The 5'-ends of all genes constructed were phosphorylated with T4 polynucleotide kinase and ATP.

<u>Construction of Recombinant Vector Plasmid</u>

A recombinat plasmid was constructed by inserting the salmon calcitonin gene or salmon calcitonin derivative gene into an HpaI-ClaI fragment of the plasmid pHT2 in such a way as that the HpaI- and ClaI-restriction enzyme recognition sequences regenerate (see Fig. 5).

The plasmid pHT2 is a plasmid which is characterized in that a Clts gene derived from pCQV2 (see C. Queen, J. Mol·Appl. Genetics 2 1, 1983) has been inserted into a vector pKC30 for expression to broaden its host range and has one restriction enzyme HpaI cleavage site in the N protein structural gene. It is possible to express a hybrid protein with N protein under an expression control by $P_L$ promoter, by inserting an extraneous gene into this cleavage site in such a manner that the trinucleotide codons of amino acids is in frame with those of N protein.

20 μg of the plasmid pHT2 was subjected to reaction in 100 μl of a ClaI reaction mixture (6 mM Tris-hydrochloride pH 7.9, 6 mM $MgCl_2$ , 50 mM NaCl) for 60 minutes at 37°C using 10 units of restriction enzyme ClaI, and DNA was precipitated by treatment with ethanol.

The DNA precipitate was dissolved in 100 μl of an HpaI reaction mixture (10 mM Tris-hydrochloride pH 7.5, 7 mM $MgCl_2$ , 100 mM KCl, 7 mM β marcaptoethanol) and was subjected to reaction for 90 minutes at 37°C after the addition of 15 units of restriction enzyme HpaI. To this was added 0.08 unit of E. coli alkaline phosphatase and reaction was carried out for 30 minutes at 65°C to dephosphorylate the 5'-end. After washing with 100 μl of phenol the reaction mixture was subjected to 0.7% agarose gel electrophoresis and large DNA fragments were electrophoretically recovered.

0.5 pmol of the resulting ClaI-HpaI DNA fragment derived from the plasmid pHT2 and 0.5 pmol of a DNA fragment containing either a salmon calcitonin structural gene or salmon calcitonin derivative structural gene having a phosphate group at the 5'-end were dissolved in 20 µl of $T_4$ DNA ligation reaction mixture (25 mM HEPES pH 7.8, 7.5 mM $MgCl_2$ , 0.2 mM ATP and 6 mM DTT) and the solution was subjected to reaction with 3 units of T4 DNA ligase for 16 hours at 20°C. Ten µl of this reaction mixtured was used to transform E. coli RR1, and transformants resistant to 100 µg/ml of ampicillin were selected. Escherichia coli RR1/pSCT1 transformed with the plasmid (pSCT1) containing the salmon calcitonin structural gene (CT1), and Escherichia coli RR1/pSCT2 transformed with the plasmid (pSCT2) containing the salmon calcitonin derivative structural gene (CT2) were thus obtained.

Base Sequence Verification of Synthesized Salmon Calcitonin and Salmon Calcitonin Derivative Genes

In order to analyze the nucleotide sequence of gene for the salmon calcitonin or the salmon calcitonin derivative in the plasmid, 15 µg each of the pSCT1 and pSCT2 plasmid DNA's was cut using 15 units of the restriction enzyme HpaI, and the 5'-end was $^{32}P$-labeled with [$^{32}P$]ATP and polynucleotide kinase after dephosphorylation using E. coli alkaline phosphatase. The intended DNA fragments were then purified after digestion with 15 units of restriction enzyme BamHI, and their nucleotide sequences were determined according to the method of Maxam and Gilbert. All the plasmids were consequently shown to have the designed nucleotide sequences.

Example 2 Construction of Plasmid pHT 31 (Figs. 6 & 15)

Five µg of plasmid pHT3 was allowed to react with 15 unit of restriction enzyme BamHI in 20 µl of a buffer (10 mM Tris-HCl pH 8.0, 7 mM $MgCl_2$ , 100 mM NaCl and 2 mM β-mercaptoethanol) for 3 hours. After precipitation with ethanol, the precipitate was reacted with one unit of the Klenow fragment in 20 µl of a buffer (50 mM Tris-HCl pH 7.2, 10 mM $MgCl_2$, 0.1 mM DTT, and 80 µM dNTP) for half an hour at 22°C. After treatment with phenol, precipitation was carried out with ethanol. The precipitate was reacted with 20 units of restriction enzyme Pvu II in 20 µl of a buffer (10 mM Tris-HCl pH 7.5, 7 mM $MgCl_2$, 60 mM NaCl, and 7 mM 2-mercaptoethanol) for 3 hours at 37°C. After precipitation with ethanol the precipitate was reacted with 2.8 units of $T_4$ DNA ligase in 20 µl of a buffer (66 mM Tris-HCl pH 7.6, 6.6 mM $MgCl_2$, 10 mM DTT, and 1 mM ATP) for 20 hours at 15°C. Escherichia coli HB101 was transformed using the reaction product. A starin containing the plasmid pHT31 shown in Fig. 15 was isolated from the ampicillin-resistant transformants.

Example 3 Construction of Plasmid pKTL1 (Figs. 7 & 16)

Thirteen µg of pST21 was digested with 18 units of HinfI in 40 µl of HinfI buffer for 3 hours at 37°C followed by precipitation with ethanol. The precipitate was reacted with 2 units of DNA polymerase I Klenow fragment in 25 µl of a nick-translation buffer containing 80 µM of dNTP for 30 minutes at room temperature. After treatment for 5 minutes at 70°C the reaction product was treated with phenol and precipitated with ethanol. The precipitate was dissolved in 40 µl of HpaI buffer and was digested with 6 units of HpaI for 1.5 hours at 37°C followed by precipitation with ethanol. The precipitate was reacted with 0.02 $OD_{260}$ units of Hind III linker in 20 µl of $T_4$ ligase buffer for 6 hours at 22°C using 350 units of $T_4$ ligase followed by precipitation with ethanol. The precipitate was digested in 40 µl of Hind III buffer for 1.5 hours at 37°C using 50 units of Hind III. the digestion product was subjected to 6% acrylamide gel electrophoresis and a 200 bp DNA fragment (1) was isolated by electrophoretic elution.

Three μg of pBR322 was digested in 40 μl of Hind III buffer for 3 hours at 37°C using 50 units of Hind III followed by precipitation with ethanol. The precipitate was dissolved in 200 μl of 50 mM Tris-HCl (pH 8.0) and subjected to reaction for 30 minutes at 65°C using 0.04 unit of alkaline phosphatase. After treatment with phenol, a DNA fragment (2) was precipitated with ethanol.

After purification using an Elutip-d (Schleicher & Schuell) column, the DNA fragment (1) (about 0.5 μg) and the DNA fragment (2) (about 3 μg) were precipitated with ethanol. The precipitate was dissolved in 20 μl of a ligase buffer and reacted with 350 units of $T_4$ ligase for 8 hours at 16°C. E. coli RR1 was transformed with 3 μl of the reaction mixture (contains about 0.6 μg of DNA). Strains which are not capable of growth in an LB agar medium containing 15 μg/ml of tetracycline were selected from among strains obtained by incubation in an LB agar medium containing 50 μg/ml of ampicillin. Plasmids were extracted from these strains and a search was made for plasmids of about 4.4 Kb in length. Plasmids of interest were further selected based on the digestion patterns by Hind III, Rsa I, Hinf I, and EcoRI/BamHI.

## Example 4 Construction of Plasmid pLMC2 (Figs. 6 & 8)

Twenty μg of pSCT2 was digested with 30 units of HpaI in 150 μl of HpaI buffer for 3 hours and the digest was precipitated with ethanol. 0.02 $OD_{260}$ unit of SalI linker d (GGTCGACC) was reacted with 4 units of $T_4$ kinase in 10 μl of kinase buffer (50 mM Tris-HCl pH 7.6, 10 mM $MgCl_2$ , 10 mM 2-mercaptoethanol) containing 10 mM ATP for one hour at 37°C. Five μl of the reaction mixture and DNA digested with HpaI were reacted with 350 units of $T_4$ ligase in 20 μl of $T_4$ ligase buffer (66 mM Tris-HCl pH 7.6, 6.6 mM $MgCl_2$ , 10 mM dithiothreitol, 0.4 mM ATP) for 5 hours at 22°C, and the reaction product was precipitated with ethanol. The precipitate was dissolved in 50 µl of SalI buffer (10 mM Tris-HCl pH 7.5, 7 mM $MgCl_2$ , 175 mM NaCl, 0.2 mM EDTA, 7 mM mercaptoethanol) and was digested with 40 units of SalI for 3 hours at 37°C. The reaction mixture was subjected to 6% acrylamide gel electrophoresis and a 600 bp DNA (1) ~~was~~ isolated electrophoretic elution.

Ten µg of pSLMK1 was digested with 40 units of SalI in 100 µl of SalI buffer for 5 hours at 37°C and the digest was precipitated with ethanol. The precipitate was dissolved in 200 µl of 50 mM Tris-HCl (pH 8.0) buffer and the solution was reacted with 0.04 unit of alkaline phosphatase for 30 minutes at 65°C, and after treatment with phenol, the reaction product was precipitated with ethanol (2).

The DNA fragment (1) (about 0.5 µg) and the DNA fragment (2) (about 1 µg) were purified on an Elutip-d (Trade Name) (Schleicher & Schuell) column and the purified fragments were precipitated with ethanol. The precipitate was dissolved in 20 µl of $T_4$ ligase buffer and the solution was reacted with 350 units of $T_4$ ligase for 5 hours at 16°C. Five µl of the reaction mixture was used to transform <u>E. coli</u> RR1. Of the transformants obtained from LB agar culture media containing 50 µg/ml of ampicillin, strains not imparting a yellow color were selected on spraying a 1% aqueous catechol solution. Plasmids were extracted from these strains and the intended plasmid pLMC2 was selected based on the digestion patterns by EcoRI, SalI, and RsaI/AvaI.

<u>Example 5 Construction of Plasmid pPMC2 (Figs. 6 & 9)</u>

Twenty µg of the pLMC2 was digested with 40 units of SalI in 200 µl of SalI buffer for 3 hours at 37°C and the digest was precipitated with ethanol. The reaction mixture was subjected to 0.7% agarose gel electrophoresis and a 600 bp DNA fragment (1) was isolated by electrophoretic elution.

Twenty μg of the pHT31 was digested with 40 units of SalI in 200 μl of SalI buffer for 3 hours at 37°C and the digest was precipitated with ethanol. The precipitate was dissolved in 200 μl of 50 mM Tris-HCl (pH 8.0) buffer and the solution was reacted with 0.04 unit of alkaline phosphatase for 30 minutes at 65°C, and after treatment with phenol, the reaction product was precipitated with ethanol. This reaction mixture was subjected to 0.7% agarose gel electrophoresis and a 5.2 Kb DNA fragment (2) was isolated by electrophoretic elution.

The DNA fragment (1) (about 3 μg) and the DNA fragment (2) (about 5.5 μg) were purified on an Elutip-d (Trade Name) (Scheicher & Schuell) and the purified fragments were precipitated with ethanol. The precipitate was dissolved in 20 ul of $T_4$ ligase buffer and the solution was reacted with 350 units of $T_4$ ligase for 24 hours at 12°C.

E. coli RR1 were transformed using 10 μl of the reaction mixture and the transformants were cultured overnight in LB agar media containing 50 μg/ml of ampicillin at 32°C. The transformants were cultured additionally for one hour at 42°C and those not imparting a yellow color on spraying a 1% aqueous catechol solution were selected. These were cultured in an LB media containing 50 μg/ml of ampicillin overnight, after which 1% of cultured broth was inoculated in a medium of the same composition and culturing was carried out further. The temperature was elevated from 32°C to 42°C at $OD_{660}$ = 0.3 and the culturing was carried out for another 3 hours. One ml of the cultured broth was centrifuged and the resulting precipitate was subjected to 12.5% SDS polyacrylamide gel electrophoresis, and strains wherein 25,000 daltons of protein were remarkably induced were selected. Plasmids were extracted from these strains and a search was made for plasmids of 5.8 Kb in length. Plasmids providing DNA fragments of the expected size were selected on the basis of the pattern of SalI digestion. These plasmids were used to transform E. coli SK383. Plasmids were extracted from the transformants and plasmids of interest were selected on the basis of the pattern of ClaI digestion.

Example 6 Construction of Plasmid pSCT12 (Figs. 7 & 10)

Five µg of plasmid DNA obtained from E. coli SK383/pSCT2 was reacted with 28 units of ClaI in 100 µl of ClaI buffer (10 mM Tris-HCl pH 7.5, 7 mM $MgCl_2$, and 7 mM 2-mercaptoethanol) for 90 minutes at 37°C and after extraction with phenol the reaction product was precipitated with ethanol. The precipitate was dissolved in 20 µl of nick translation buffer (50 mM Tris-HCl pH 7.2, 10 mM $MgSO_4$, and 1 mM dithiothreitol) containing 0.2 mM each of dGTP, dATP, TTP, and dCTP, and after addition of 5 units of E. coli DNA polymerase I large fragment, the solution was subjected to reaction for 30 minutes at 22°C. The reaction product was extracted with phenol and the extracts were precipitated with ethanol. The precipitate was dissolved in 20 µl of ligase buffer together with 0.02 OD of activated SalI linker and the mixture was reacted with 700 units of $T_4$ DNA ligase for 12 hours at 12°C. The reaction product was precipitated with ethanol and the precipitate was dissolved in 50 µl of SalI buffer. The solution was reacted with 24 units of SalI for 120 minutes at 37°C. The reaction product was subjected to heat treatment for 10 minutes, and thereafter, was precipitated with ethanol. The precipitate was dissolved in 200 µl of ligase buffer and the solution was reacted with 170 units of $T_4$ DNA ligase for 5 hours at 22°C. Fifty µl of the fraction mixture was used to transform strain HB101. Transformants were obtained from LB agar media containing 50 µg/ml of ampicillin and plasmids were extracted from them by the Holmes & Quigley Method (D.S. Holmes & M. Quigley, Anal. Biochem. Vol. 114, 193-197, 1981). The intended plasmid pSCT12 was selected from among these plasmids on the basis of the patterns of SalI digestion and SalI-HpaI double digestion.

Example 7 Construction of Plasmid pUCT12 (Figs. 7 & 11)

(i) Ten μg of the pUC9 was dissolved in 100 μl of Hind III buffer (10 mM Tris-HCl pH 7.5, 7 mM $MgCl_2$, and 60 mM NaCl) and the solution was allowed to digest with 35 units of Hind III for 2 hours at 37°C. The digestion product was then precipitated with ethanol.

(ii) To the precipitate was added 20 μl of nick translation buffer and the solution was allowed to react with 2 μl of a solution containing 2 mM each of dGTP, dATP, dCTP, and TTP and 2.5 units of E. coli DNA polymerase I Klenow fragment for 30 minutes at 22°C. The reaction mixture was extracted with phenol and the product was precipitated with ethanol.

(iii) To the precipitate were added 20 μl of ligase buffer (66 mM Tris-HCl, 6.6 mM $MgCl_2$, 0.4 mM ATP, pH7.6) and 0.02 OD of activated XhoI linker d(CCTCGAGG). The mixture was allowed to react with 350 units of $T_4$ DNA ligase for 12 hours at 12°C.

(iv) The reaction product was precipitated with ethanol and the precipitate was dissolved in 100 μl of SalI buffer. The solution was allowed to react with 25 units each of SalI and XhoI for 4 hours at 37°C.

(v) To the reaction product was added 100 μl of 50 mM Tris-HCl buffer (pH 8.0) and 0.04 unit of E. coli alkaline phosphatase, and reaction was carried out for 30 minutes at 65°C. Extraction with phenol was repeated twice and precipitation with ethanol was carried out and the precipitate was dissolved in 200 μl of TE buffer (10 mM Tris-HCl, 1 mM EDTA, pH 8.0).

(vi) Twenty μg of the pSCT12 was dissolved in 100 μl of HpaI buffer and the solution was added with 35 units of HpaI, and reaction was carried out for 4 hours at 37°C to digest the plasmid. The digestion product was then precipitated with ethanol.

(vii) The addition of XhoI linker to the digestion product, and SalI and XhoI digestion thereof were carried out by the methods described in paragraphs (iii) and (iv) above. The entire amount of the digest was subjected to 6% polyacrylamide gel electrophoresis, and after staining with ethidium bromide, DNA fragments measuring about 120 bp in length were recovered by electrophoretic elution.

(viii) Fifty µl of the DNA solution obtained as described in paragraph (v) was added to the recovered fragment solution, and after treatment on an Elutip-d column (Schleicher & Schuell), DNA was precipitated with ethanol.

(ix) The precipitate was added with 20 µl of ligase buffer and 80 units of $T_4$ DNA ligase, and reaction was carried out for 10 hours at 16°C.

(x) Five µl of the reaction mixture was used to transform E. coli HB101 and colonies capable of growth in an LB media (1% bactotrypton, 0.5% yeast extract, 1% NaCl pH 7.4, 1.5% agar) containing 50 µg/ml of ampicillin were obtained.

(xi) Plasmid DNA's were extracted from the transformants by the Holmes & Quigley method described earlier and a plasmid corresponding to pUCT12 in size and restriction enzyme cleavage pattern was selected.

## Example 8 Construction of Plasmid pUCT22 (Figs. 7 & 11)

Ten µg of the pUCT12 was dissolved in 100 µl of EcoRI buffer (50 mM Tris-HCl pH 7.5, 7 mM $MgCl_2$, 100 mM NaCl, 7 mM 2-mercaptoethanol) and the solution was added with 22 units of EcoRI, and reaction was carried out for 2 hours at 37°C to digest the plasmid. The digest was precipitated with ethanol and the precipitate was dissolved in 100 µl of BamHI buffer (10 mM Tris-HCl pH 8.0, 7 mM $MgCl_2$, 100 mM NaCl, 2 mM 2-mercaptoethanol) and the solution was added with 24 units of BamHI, and digestion was carried out by reaction for 2 hours at 37°C. This EcoRI-BamHI double-digest was subjected to dephosphorylation at the 5'-end as described in Example 7, paragraph (v), and precipitated with ethanol (1).

Thirty µg of the pKTL1 was dissolved in 100µl of EcoRI buffer and the solution was added with 90 units of EcoRI and reaction was carried for 4 hours at 37°C to digest the plasmid. The digest was precipitated with ethanol. The precipitate was dissolved in 100 µl of BamHI buffer and the solution was added with 72 units of BamHI, and digestion was carried out by reaction for 2 hours at 37°C. The digest was precipitated with ethanol. The digest was subjected to 1% agarose gel electrophoresis (50 mM Tris-borate, 1 mM EDTA, pH 8.3), and after staining with ethidium bromide, DNA fragments measuring about 600 bp in length were recovered by electrophoretic elution. After treatment on an Elutip-d column the recovered fragments were precipitated with ethanol and the precipitate was dissolved in 50 µl of ligase buffer.

Twenty-five µl of the fragment solution was added to the DNA digest (1) and mixed, and the mixture was added with 80 units of $T_4$ DNA ligase, and reaction was carried out for 6 hours at 15°C.

Five µl of the reaction product was used to transform E. coli HB101 and colonies capable of growth on LB agar medium containing 50 µg/ml of ampicillin were obtained. Plasmid DNA's were extracted from the transformants and plasmid corresponding to the pUCT22 in size and restriction enzyme cleavage pattern was selected.

Example 9 Construction of Plasmid pTCCMAX (Figs. 7 & 12)

Five μg of pTCCM1 was dissolved in 50 μl of AvaI buffer (10 mM Tris-HCl pH 8.0, 7 mM $MgCl_2$, 60 mM NaCl, 7 mM 2-mercaptoethanol) and the solution was added with 20 units of AvaI, and reaction was carried out for 2 hours at 37°C to digest the plasmid. The digest was precipitated with ethanol. The precipitate was dissolved in 20 μl of nick translation buffer. The solution was added with 2 μl of a solution containing 2 mM each of dGTP and dCTP, and 2.5 units of E. coli DNA polymerase Klenow fragment, and reaction was carried out for 30 minutes at 22°C, and the DNA was precipitated with ethanol. The precipitate was dissolved in 200 μl of S1 buffer (30 mM sodium acetate pH 4.6, 50 mM NaCl, 1 mM $ZnSO_4$, 50% glycerol) and the solution was added with 3 units of S1 nuclease, and reaction was carried out for 10 minutes at 37°C. The reaction mixture was extracted with phenol and precipitated with ethanol. The addition of XhoI linker was then carried out by the method described in Example 7, Paragraphs (ii) and (iii). This reaction product was used to transform E. coli JM103 and colonies capable of growth on an LB agar medium containing 50 μg/ml of ampicillin were obtained. Plasmid DNA's were extracted from the transformants and plasmid corresponding to pTCCMAX in size and restriction enzyme cleavage pattern was selected.

Example 10 Constructoin of Plasmid pTMC2 (Figs. 7 & 12)

(i) Ten μg of the pUCT22 was dissolved in 50 μl of XhoI buffer (10 mM Tris-HCl pH 7.5, 7 mM $MgCL_2$, 100 mM NaCl, 7 mM 2-mercaptoethanol) and the solution was added with 25 units of XhoI, and reaction was carried out for 2 hours at 37°C. The reaction product was precipitated with ethanol.

(ii) The treatment with Klenow fragments was carried out by the same technique as that described in Example 7, Paragraph (ii) and the reaction product was precipitated with ethanol.

(iii) The precipitate was mixed with 20 µl of ligase buffer and 0.05 OD of activated HpaI linker and added with 350 units of $T_4$ DNA ligase, and reaction was carried out for 10 hours at 12.5°C.

(iv) The reaction product was precipitated with ethanol and the precipitate was dissolved in 50 µl of HpaI buffer. The solution was added with 18 units of HpaI and 24 units of Ban III, and reaction was carried out for 2 hours at 37°C.

(v) The total amount of the digest was subjected to 1% agarose gel electrophoresis, and after staining with ethidium bromide, DNA fragments measuring about 690 bp in length were recovered by electrophoretic elution.

(vi) Five µg of the pTCCMAX was dissolved in 50 µl of XhoI buffer and the solution was added with 25 units of XhoI, and reaction was carried out for 2 hours at 37°C. The reaction product was precipitated with ethanol.

(vii) The treatment with Klenow fragments was carried out by the same technique as that described in Example 7, paragraph (ii).

(viii) The addition of HpaI linker to and digestion of this linear DNA were carried out by the same method as those described in paragraphs (iii) and (iv).

(ix) Half of the digests were subjected to 1% agarose gel electrophoresis, and after staining with ethidium bromide, DNA fragment measuring about 3.4 Kb was recovered by electrophoretic elution.

(x) The resulting DNA solution was mixed with the DNA obtained in Paragraph (v), and after treatment on an Elutip-d column, the product was precipitated with ethanol.

(xi) The precipitate was added with 20 µl of ligase buffer and 170 units of $T_4$ DNA ligase, and reaction was carried out for 8 hours at 16°C.

(xii) Half of the reaction products were subjected to 0.7% agarose gel electrophoresis, and after staining with ethidium bromide, DNA fragment measuring about 4.1 Kb in length was recovered by electrophoretic elution.

(xiii) The recovered DNA was used to transform E. coli JM103 and colonies capable of growth on an LB agar medium containing 50 µg/ml of ampicillin were obtained. Plasmid DNA's were extracted from the transformants and a plasmid measuring about 4.1 Kb was selected.

Example 11 Construction of Plasmid pTMC12 (Fig. 7)

Three µg of pTMC2 was digested with 10 units of XhoI in 100 µl of XhoI buffer (10 mM Tris-HCl pH 7.5, 7 mM $MgCl_2$, 100 mM NaCl, 7 mM 2-mercaptoethanol) for 15 hours at 37°C. The digest was precipitated with ethanol and the precipitate was dissolved in a nick translation buffer containing 0.2 mM each of dGTP, dATP, TTP and dCTP. The solution was added with 2 units of DNA polymerose I Klenow fragment, and reaction was carried out for 30 minutes at 22°C. After phenol extraction and ethanol precipitation, the precipitate was dissolved in 20 µl of ligase buffer together with 1 µg of HpaI linker dGTTAAC, and the solution was added with 700 units of $T_4$ DNA ligase, and reaction was carried out for 8 hours at 22°C. The reaction mixture was used to transform E. coli JM103 and a transformant containing the pTMC12 was selected.

Example 12 Construction of Plasmid pTMC22 (Figs. 7 & 13)

Twenty µg of the pTCCMAX was digested with 60 units of XhoI and 50 units of Hind III in 100 µl of Hind III buffer for 3 hours at 37°C. The digest was subjected to polyacrylamide gel electrophoresis and the DNA fragment measuring about 640 bp was isolated by electrophoretic elution. The fragment was precipitated with ethanol, and the precipitate was digested with 16 units of Sau 3AI in 100 µl of Sau 3AI buffer (10 mM Tris-HCl pH 7.5, 7 mM $MgCl_2$ , 100 mM NaCl) for 2 hours. The digest was precipitated with ethanol and the precipitate was dissolved in 20 µl of nick translation buffer containing 0.2 mM each of dGTP, dATP, TTP, and dCTP. The solution was added with 2 units of DNA polymerase I Klenow fragment, and reaction was carried out for 30 minutes at 22°C. After phenol extraction and ethanol precipitation, the precipitate was dissolved in 20 µl of ligase buffer together with 1 µg of activated HpaI linker, and the solution was added with 700 units of $T_4$ DNA ligase, and reaction was carried out for 12 hours at 12°C. After ethanol precipitation, the reaction product was dissolved in 50 µl of HpaI buffer (10 mM Tris-HCl pH 7.5, 7 mM $MgCl_2$ , 100 mM KCI, 7 mM 2-mercaptoethanol) and the product was digested with 18 units of HpaI for 2 hours at 37°C. After precipitation with ethanol, the reaction product was dissolved in 50 µl of EcoRI buffer (50 mM Tris-HCl pH 7.5, 7 mM $MgCl_2$, 100 mM NaCl, 7 mM 2-mercaptoethanol) and the solution was subjected to digestion with 20 units of EcoRI for 2 hours at 37°C. The digest was subjected to polyacrylamide gel electrophoresis and the DNA fragment (fragment A) measuring about 240 bp was isolated by electrophoretic elution.

Ten µg of the pTMC12 was digested with 45 units of PstI in 100 µl of PstI buffer (10 mM Tris-HCl pH 7.5, 7 mM $MgCl_2$ , 100 mM NaCl, 7 mM 2-mercaptoethanol) for 2 hours at 37°C. Five µg of the resulting PstI digest of the pTMC12 was precipitated with ethanol and the precipitate was digested with 30 units of EcoRI in 100 µl of EcoRI buffer for 3 hours at 37°C. After ethanol precipitation, the precipitate was dissolved in 100 µl of ClaI buffer (10 mM Tris-HCl pH 7.5, 7 mM $MgCl_2$ , 7 mM 2-mercaptoethanol) and digested with 20 units of ClaI for 2 hours at 37°C. The digest was subjected to agarose gel electrophoresis and a DNA fragment (fragment B) measuring about 1500 bp in length was isolated by electrophoretic elution.

Five μg of the PstI-digest of the pTMC12 was digested with 24 units of HpaI in 100 μl of HpaI buffer for 3 hours at 37°C. The digest was subjected to agarose gel electrophoresis and a DNA fragment (fragment C) measuring about 1800 bp in length was isolated by electrophoretic elution.

The DNA fragments A, B and C were mixed, treated on an Elutip-d (Trade Name) column, and precipitated with ethanol. The precipitate was dissolved in 20 μl of ligase buffer and the solution was added with 700 units of $T_4$ DNA ligase and reaction was carried out for 12 hours at 12°C. The reaction product was used to transform E. coli JM103 and a transformant carrying the pTMC22 was selected.

Example 13 Construction of Plasmid pTMC32 (Figs. 7 & 14)

Twenty μg of the pTMC12 was digested with 30 units of Aat II in 100 μl of Aat II buffer (10 mM Tris-HCl pH 7.5, 7 mM $MgCl_2$ , 60 mM KCl, 7 mM 2-mercaptoethanol) for 2.5 hours at 37°C. The digest was subjected to polyacrylamide gel electrophoresis and a DNA fragment measuring about 400 bp in length was isolated by electrophoretic elution. This DNA fragment was digested with 24 units of AluI in 100 μl of AluI buffer (10 mM Tris-HCl, 7 mM $MgCl_2$ , 20 mM NaCl, 7 mM 2-mercaptoethanol) for 3 hours at 37°C. The digest was subjected to polyacrylamide gel electrophoresis and a DNA fragment (fragment A) measuring about 245 bp in length was isolated by electrophoretic elution.

Ten μg of the pTMC22 was digested with 45 units of PstI in 100 μl of PstI buffer for 16 hours at 37°C. Five μg of the resulting PstI-digest of the pTMC22 was precipitated with ethanol and the precipitate was digested with 30 units of Pvu II in 100 μl of Pvu II

buffer (10 mM Tris-HCl pH 7.5, 7 mM $MgCl_2$ , 60 mM NaCl, 7 mM 2-mercaptoethanol) for 2 hours at 37°C. The digest was subjected to agarose gel electrophoresis, and a DNA fragment (fragment B) measuring about 1800-bp in length was isolated by electrophoretic elution.

Five µg of the PstI-digest of the pTMC22 was digested with 24 units of HpaI in 100 µl of HpaI buffer for 2 hours at 37°C.

After ethanol precipitation, the precipitate was dissolved in 100 µl of NdeI buffer (10 mM Trist-HCl pH 7.5, 7 mM $MgCl_2$ , 175 mM NaCl, 7 mM 2-mercaptoethanol), and digested with 18 units of NdeI for 9 hours at 37°C. The digest was subjected to agarose gel electrophoresis, and a DNA fragment (fragment C) measuring about 1820 bp in length was isolated by electrophoretic elution. The DNA fragments A, B, and C were mixed, and after treatment on an Elutip-d (Trade Name) column, precipitated with ethanol, and ligated with 350 units of $T_4$ DNA ligase in 20 µl of ligase buffer for 17 hours at 12°C. The ligation product was used to transform E. coli JM103 and a transformant carrying the pTMC32 was selected.

Example 14 Introduction of Site Directed Mutation Using M13 Phage (Construction of Eel Calcitonin Derivative Gene) (Fig. 17)

(1) Preparation of Single-stranded Template DNA

0.5 pM of an HpaI-SalI 0.6 Kbp DNA fragment containing a salmon calcitonin derivative gene derived from the plasmid pSCT2 carrying the salmon calcitonin derivative gene and 0.5 pM of SmaI-SalI 2.7 Kbp DNA fragment derived from the double-stranded DNA of M13 phage mp 9 were mixed, and the mixture was ligated with 100 units of $T_4$ DNA ligase in 20 µl of a solution containing 66 mM Tris-HCl (pH 7.5), 5 mM

$MgCl_2$ , 5 mM DTT and 1 mM ATP for 16 hours at 12°C. The reaction mixture was used to transform E. coli JM103 according to the method described by Messing (Methods in Enzymology, 101, 20-78, 1983). A soft agar plate medium containing 0.02% X-gal and 1 mM IPTG was inoculated with the transformed cells and was incubated overnight at 37°C. A single-stranded template DNA was prepared from a white plaque formed by the transformant. That is, a white plaque was picked up with the tip of a toothpick, and suspended in 1.5 ml of a 2xYT medium (1.6% bactotrypton, 1% yeast extract, and 0.5% NaCl) in which the E. coli JM103 cells are growing, and culturing was carried out for 5 hours at 37°C. From a supernatant of the cultured broth, a single-stranded recombinant phage DNA was recovered by polyethyleneglycol precipitation, phenol treatment, and ethanol precipitation.

Using the resulting single-stranded DNA as a template, the base sequence was determined by the method of Messing et al (described earlier) and the sequence of the cloned single-stranded DNA was confirmed. The single-stranded DNA containing an anticoding chain of the salmon calcitonin derivative gene was thus obtained. This recombinant phage was designated as CTM31.

(2) Synthesis of Double-stranded DNA Using Oligonucleotide as Primer Using the resulting single-stranded DNA of the CTM31 as a template, repair-reaction by the DNA polymerase Klenow fragment was carried out with the synthesized oligonucleotide:

5' TGGTCGTGGTTGCAGCCATGCGC 3' serving as a primer. That is, 2 pM of the primer with the phosphorlated 5'-end was added to 0.5 pM of the single-stranded template DNA, and the mixture was allowed to stand in 10 µl of a solution containing 7 mM Tris-HCl (pH 7.5), 0.1 mM EDTA, 20 mM NaCl, and 7 mM $MgCl_2$ first for 20 minutes at 60°C and then for another 20 minutes at 23°C. This reaction mixture was added with dATP, dGTP, TTP and dCTP, each to the concentration of 0.5 mM, and added with a DNA polymerase Klenow fragment to a final volume of 20 μl. The mixture was first incubated for 20 minutes at 23°C, and then after the addition of 1 μl of 10 mM ATP and 1 unit of $T_4$ DNA ligase, incubated overnight at 12°C.

(3) Isolation of Double-stranded DNA by Agarose Gel Electrophoresis

In order to eliminate the unreacted single-stranded DNA serving as a background from the resulting double-stranded DNA, the total amount of the reaction mixture was subjected to separation by 0.8% agarose gel electrophoresis containing 2 μg/ml of ethidium bromide. After identifying the intended double-stranded DNA, a piece of gel was cut out from the corresponding portion and was placed in a dialysis tube, which was sealed after having been filled with 89 mM Tris-boric acid-2mM EDTA buffer. The DNA in the gel was eluted by electrophoresis performed for 12 hours in the buffer at a constant voltage of 50 V. The DNA was then withdrawn from the dialysis tube and was lyophilized. The lyophilizate was reconstituted in 100 μl of distilled water and purified on a disposable ion exchange column Elutip-d (Scheicher & Schuell).

(4) Transformation of E. coli JM103

E. coli JM103 was transformed by the method of Messing with 0.1 pM of the double-stranded DNA.

(5) Detection of Mutants

Using the phage plaques, mutant phages were screened by plaque hybridization using as a probe the synthesized oligonucleotide labeled with $^{32}P$:

5' TCGTGGTTGCAGCCA 3'

That is, according to the method of Benton and Davis (W.D. Benton and R.W. Davis, Science, 196, 180, 1977), the plaques were transferred from the soft agar medium to a nitrocellulose filter, and the filter was baked in vacuo for 2 hours at 80°C. This nitrocellulose filter was subjected to hybridization in a 6 x SSC and 10 x Denhardt solution overnight at 23°C using the $^{32}P$-labeled primer oligonucleotide as a probe. The filter was then washed in a 6 x SSC solution at 46°C and mutant phage plaques showing a positive signal were isolated by autoradiography.

(6) Determination of Nucleic Acid Sequence of the Mutant DNA

The nucleic acid sequence was determined by the dideoxy chain termination method using the mutant phage DNA as a template and it was confirmed that base substitution mutation had taken place and the phage carrying the eel calcitonin derivative gene had been obtained. This mutant phage plaque strain was designated as JM103/CTM41.

Example 15 Construction of Plasmid pCTM4 (Fig. 18)

Ten pM of double-stranded DNA of phage CTM41 was digested with 20 units of EcoRI in 100 µl of buffer containing 10 mM Tris-HCl (pH 7.5), 0.7 mM $MgCl_2$, 5 mM NaCl and 0.7 mM β-mercaptoethanol for 4 hours at 37°C. The reaction mixture was extracted with phenol and chloroform, and after precipitation of DNA with ethanol, the precipitate was dried. This DNA was filled-in with 20 units of DNA polymerase Klenow fragment in 100 µl of buffer containing 50 mM Tris-HCl (pH 8.0), 75 mM NaCl, 10 mM $MgCl_2$, 5 mM β-mercaptoethanol, and 0.25 mM each dATP, dGTP, dCTP, TTP and ATP for 2 hours at 37°C. The reaction mixture was extracted with phenol and chloroform, and after precipitation of DNA with ethanol the precipitate was dried. This DNA was digested with 8 units of SalI in 100 µl of buffer containing 1 mM Tris-HCl (pH 7.5), 0.7 mM $MgCl_2$, 15 mM NaCl, 0.7 mM β-mercaptoethanol and 0.02 mM EDTA for 2 hours at 37°C. The reaction mixture was electrophoretically separated on 1.2% agarose gel containing ethidium bromide and a portion of the gel containing small DNA fragment was cut out. The intended DNA fragment was eluted by the electric elution using a dialysis membrane. A DNA fragment was precipitated from the eluate with ethanol and the precipitate was dried. The dried material was dissolved in 40 μl of water.

Ten pM of DNA of plasmid pTCCM1 was digested with 20 units of AvaI in 100 μl of a buffer containing 10 mM Tris-HCl (pH 7.4), 7 mM $MgCl_2$, 50 mM NaCl and 0.1 mM EDTA for 4 hours at 37°C. The reaction mixture was extracted with phenol and chloroform, and DNA was precipitated with ethanol, and the DNA was dried. Then, the DNA was subjected to fill-in reaction, digestion with SalI, and the gel separation as described for the CTM41.

Five μl each of the solutions containing the DNA fragments were subjected to the ligation using 30 units of $T_4$ DNA ligase in a total amount of 100 μl of buffer containing 25 mM N-2-hydroxyethylpiperazine-N'-2-ethanesulfonic acid (HEPES) (pH 7.8), 7 mM $MgCl_2$, 12 mM dithiothreitol, and 0.4 mM ATP for 24 hours at 16°C.

Ten μl of the reaction mixture was used to transform E. coli JM103.
Colony hybridization was carried out on E. coli colonies thus treated using the calcitonin derivative structural gene U31 fragment (GGGAAGTTGAGTCAG) (see Example 1 and Fig. 1) and positive colonies were obtained at a frequency of about 60%. Expression tests were run on these positive clones and a clone capable of expressing protein of about 19,000 daltons was selected. Plasmid DNA was prepared from the selected clone and the base sequence was determined by dideoxy sequencing method using the calcitonin derivative structural gene L-41 fragment (GTAATTCCTGACTCA) (see Example 1 and Fig. 1) as a probe, thereby confirming the proper insertion of the eel calcitonin derivative gene. This plasmid was designated as pCTM4.

The plasmid contained the gene coding for fused protein of the metapyrocatechase and the eel calcitonin derivative under the control of the tac promoter.

Example 16 Preparation of Salmon Calcitonin Derivative

(1) Culturing

E. coli RB791 containing the plasmid pTMC2 was cultured overnight in 10 ml of LB medium containing 50 µg/ml of ampicillin at 37°C with shaking. One liter of the same medium was then inoculated with the cultured broth, and after culturing for 2 hours, was added with IPTG to make the final concentration 1 mM, and cultured for an additional 6 hours.

(2) Isolation of Fused Protein

The culture broth thus obtained was centrifuged, and the precipitate was washed with 50 mM potassium phosphate buffer (pH 7.5) to obtain about 9.5 g of the cells.

The cells were suspended in 50 ml of 50 mM potassium phosphate buffer containing 10 mM EDTA, and the suspension was added with 100 mg of lysozyme, allowed to stand in ice for 30 minutes, and then subjected to ultrasonic treatment for 30 minutes to disrupt the cells. The treated mixture was centrifuged for 30 minutes at 15000 rpm to obtain an insoluble fraction, the fraction was suspended in 50 ml of aqueous 7M guanidine hydrochloride solution, and the suspension was allowed to stand at 0°C for 30 minutes to solubilize the fused protein. The aqueous solution thus prepared was dialysed overnight against 2 ℓ of distilled water to form an insoluble white precipitate of the fused protein. The dialysate was centrifuged and the precipitate was recovered as a fused protein fraction. The recovered precipitate was dissolved in an aqueous 4M guanidine hydrochloride solution and was subjected to Sephadex G75 column chromatography. This column was 2.5 cm in inner diameter, 45 cm in height, and 220 ml in bed volume. Elution was carried out with an aqueous 4M guanidine hydrochloride solution. Figure 19 shows the elution pattern (A) and the gel pattern (B) of each fraction on SDS polyacrylamide gel electrophoresis. A fused protein was eluted as an almost single band in the fraction corresponding to about 20,000 daltons.

Fractions from the exclusion volume fraction to the fused protein fraction (from the fraction No. 17 through the fraction No. 20) in the column chromatography were recovered and subjected to treatment in the next step.

(3) Isolation of Salmon Calcitonin Derivative

After overnight dialysis against 2 ℓ of distilled water, the resultant fused protein fraction was centrifuged for 30 minutes at 14,000 rpm. The fused protein was cleaved into the metapyrocatechase and the salmon calcitonin derivative by treating the resulting precipitate with CNBr in the following manner: Twenty ml of 70% formic acid and 500 mg of CNBr were added to the precipitate and the mixture was subjected to reaction for 24 hours at the ambient temperature in a dark place. At the end of the reaction period, 180 ml of water was added and a white powder was obtained on lyophilization. The powder was dissolved in 0.1% trifluoroacetic acid (TFA) and the solution was fractionated by HPLC using an RP304 column (manufactured by Bio-Rad Inc.). Elution was carried out using a linear gradient of 26% to 40% acetonitrile aqueous solution in 0.1% TFA. The elution profile is shown in Fig. 20A. Thus a peak fraction having a retention time closely coresopnding to that of the commercially available salmon calcitonin I was separated. The resulting fraction was subjected to gel filtration HPLC on a TSKG2000SW column (manufactured by Toyo Soda Co.) in 0.05% aqueous TFA solution for further purification and a peak having a molecular weight corresponding to that of the commercially available salmon calcitonin was obtained (Fig. 20B). The degree of purification of the collected sample was evaluated on the basis of the elution patterns generated respectively by HPLC on the RP304 column and by HPLC on the TSKG2000SW column. Both HPLC's showed a single peak having the retention time closely corresponding to that of the commercially available salmon calcitonin I.

Example 17 Preparation of Eel Calcitonin Derivative

(1) Culturing

*E. coli* JM103 containing the plasmid pCTM4 was cultured overnight in 10 ml of LB medium containing 50 µg/ml of ampicillin at 37°C with shaking. One liter of the same medium was inoculated with 10 ml of the cultured broth and culturing was carried out for 6 hours at 37°C. When the $OD_{550}$ reached about 0.5, IPTG was added to the culturing broth to make the final concentration 1 mM, and further culturing was carried out for another 6 hours.

(2) Isolation of Fused Protein

The cells were harvested by centrifugation of the broth thus obtained for 15 minutes at 6000 rpm. The cells were added with 50 mg of lysozyme and suspended in 50 mM potassium phosphate buffer (pH 7.5, containing 10 mM EDTA) to make 50 ml. The suspension was allowed to stand at 0°C for 30 minutes and the cells were disrupted by ultrasonic treatment conducted twice for each 15 minutes. The suspension was separated into the supernatant (Fig. 21, S-1) and the insoluble fraction (Fig. 21, P-1) on centrifugation for 30 minutes at 10,000 rpm and at 4°C. As illustrated in Fig. 21, SDS-polyacrylamide gel electrophoretic analysis showed the presence of most of protein in the insoluble fraction. This insoluble fraction was resuspended in 50 ml of potassium phosphate buffer, in the same way as aforementioned, and the suspension was separated into the supernatant (Fig. 21, S-2) and the insoluble fraction (Fig. 21, P-2) on centrifugation for 30 minutes at 10,000 rpm and at 4°C. As illustrated in Fig. 21, the bulk of protein containing about 19,100 daltons of the intended fused protein was present in the insoluble fraction. This insoluble fraction was suspended in 50 ml of aqueous 7M guanidine hydrochloride solution and the suspension was allowed to stand at 0°C for 30 minutes to solubilize the fused protein. The contents of this solubilized fraction were mainly the desired fused protein (Fig. 21, SP-3). This fraction was centrifuged for 15 minutes at 8,000 rpm to give the supernatant containing the fused protein. The fused protein was precipitated by dialyzing the supernatant against distilled water to eliminate the guanidine hydrochloride. With the addition of about 50 ml of distilled water the precipitate was lyophilized and about 280 mg of a pale yellow powder was obtained.

(3) Isolation of Eel Calcitonin Derivative

Twenty ml of 70% formic acid was added to the powder, followed by the addition of 500 mg of CNBr, and the fused protein was cut to liberate the eel calcitonin derivative peptide from the metapyrocatechase protein by allowing the mixture to stand overnight at the ambient temperature and in a dark place. This mixture was then diluted with water and lyophilized. About 260 mg of a white powder was obtained.

With the addition of 20 ml of 0.1% trifluoroacetic acid (TFA) the lyophilized powder was chromatographically treated on a C18 column. Fractions were obtained by linear gradient elution in the 0.1% TFA with 10%-50% $CH_3CN$. This elution profile is shown in Fig. 22. The collected fractions were analyzed by reversed phase high performance liquid chromatography (reversed-phase HPLC) on an RP-304 column and the fractions (represented by the arrow in Fig. 22) having a retention time very similar to that of the salmon calcitonin I preparation were obtained. These fractions were purified in 0.05% TFA by gel filtration HPLC using a TSK-G2000SW column. This purification profile is illustrated in Fig. 23. Fractions having a molecular weight closely corresesopnding to that of the salmon calcitonin I preparation were collected and purified twice by reversed-phase HPLC on an RP-304 column. There were obtained fractions having a molecular weight closely corresponding to that of the salmon calcitonin I preparation. The profile of the second reversed-phase HPLC is illustrated in Fig. 24. Analysis of these fractions containing the eel calcitonin derivative by RP304 reversed-phase HPLC and TSK-G2000SW gel filtration HPLC confirmed, as shown in Fig. 25 (A) and Fig. 25 (B), that a single peak representing the molecular weight closely corresponding to that of the salmon calcitonin I preparation had been obtained and the homogeneous eel calcitonin derivative had been obtained. About 500 μg of eel calcitonin derivative was obtained in the form of a white powder by lyophilization of the uniform fractions.

CAPABILITY OF EXPLOITATION IN INDUSTRY

The process of the present invention permits the fish calcitonin derivative to be prepared economically on a large scale. The fish calcitonin derivative thus prepared may be used as a pharmaceutical in the same manner as other calcitonins. Furthermore, the calcitonin derivative of the present invention may be used as an intermediate for the preparation of calcitonins of the natural type.

CLAIMS

1. A structural gene for salmon calcitonin.

2. A gene according to claim 1 wherein said structural gene consists of the following base sequence:

Code Chain: T G C T C C A A T C T C T C T A C T -
Anticode Chain: A C G A G G T T A G A G A G A T G A -
T G C G T T C T G G G G A A G T T G A G T -
A C G C A A G A C C C C T T C A A C T C A -
C A G G A A T T A C A T A A G C T G C A A -
G T C C T T A A T G T A T T C G A C G T T -
A C T T A C C C G C G T A C C A A C A C T -
T G A A T G G G C G C A T G G T T G T G A -
G G T T C T G G T A C A C C T
C C A A G A C C A T G T G G A

3. A process for production of a structural gene of salmon calcitonin comprising the steps of:

(1) synthesizing a plurality of oligonucleotides consisting a portion of the base sequence of said structural gene,

(2) constructing a plurality of subblocks by connecting said oligonucleotides, and

(3) connecting said subblocks.

4. A plasmid containing a structural gene of salmon calcitonin

5. A plasmid according to claim 4 which is plasmid pSCT1.

6. <u>Escherichia coli</u> transformed by plasmid containing a structural gene of salmon calcitonin.

7. <u>E. coli</u> according to claim 6 which is <u>Escherichia coli</u> RR1/pSCT1 (FERM-P7774).

8. A gene coding for a fish calcitonin derivative represented by the following amino acid sequence (I):

Cys Ser Asn Leu Ser Thr Cys Val Leu Gly
Lys Leu Ser Glu Glu Leu His Lys Leu Gln
Thr Tyr Pro Arg Thr X Y Gly Z Gly
Thr Pro Gly

wherein X represents Asn or Asp, Y represents Thr or Val, and Z represents Ser or Ala.

9. A gene according to claim 8 coding for a salmon calcitonin derivative represented by the following amino acid sequence (II):

Cys Ser Asn Leu Ser Thr Cys Val Leu Gly
Lys Leu Ser Gln Glu Leu His Lys Leu Gln
Thr Tyr Pro Arg Thr Asn Thr Gly Ser Gly
Thr Pro Gly.

10. A gene according to claim 9 having the following base sequence:

Code Chain: T G C T C C A A T C T C T C T A C T -
Anticode Chain: A C G A G G T T A G A G A G A T G A -
T G C G T T C T G G G G A A G T T G A G T -
A C G C A A G A C C C C T T C A A C T C A -
C A G G A A T T A C A T A A G C T G C A A -
G T C C T T A A T G T A T T C G A C G T T -
A C T T A C C C G C G T A C C A A C A C T -
T G A A T G G G C G C A T G G T T G T G A -
G G T T C T G G T A C A C C T G G T
C C A A G A C C A T G T G G A C C A

11. A gene coding for an eel calcitonin derivative represented by the following amino acid sequence (III):

Cys Ser Asn Leu Ser Thr Cys Val Leu Gly
Lys Leu Ser Gln Glu Leu His Lys Leu Gln
Thr Tyr Pro Arg Thr Asp Val Gly Ala Gly
Thr Pro Gly.

12. A gene according to claim 11 having the following base sequence:

Code Chain: T G C T C C A A T C T C T C T A C T -
Anticode Chain: A C G A G G T T A G A G A G A T G A -
T G C G T T C T G G G G A A G T T G A G T -
A C G C A A G A C C C C T T C A A C T C A -
C A G G A A T T A C A T A A G C T G C A A -
G T C C T T A A T G T A T T C G A C G T T -
A C T T A C C C G C G T A C C G A C A T T -
T G A A T G G G C G C A T G G C T G C A A -

G G T G C T G G T A C A C C T G G T
C C A C G A C C A T G T G G A C C A

13. A DNA fragment coding for a fused protein of the fish calcitonin derivative represented by the following amino acid sequence (I):

Cys Ser Asn Leu Ser Thr Cys Val Leu Gly
Lys Leu Ser Gln Glu Leu His Lys Leu Gln
Thr Tyr Pro Arg Thr X Y Gly Z Gly
Thr Pro Gly

wherein X represents Asn or Asp, Y represents Thr or Val, and Z represents Ser or Ala, and other protein.

14. A DNA fragment according to claim 13 consisting of a gene coding for said fish calcitonin derivative and a metapyrocatechase gene or a portion thereof located upstream from fish calcitonin derivatives gene with said genes being interrupted by methionine coton ATG.

15. A DNA fragment according to claim 14 wherein said metapyrocatechase gene or a portion thereof consists of the SD sequence of the metapyrocatechase gene and the whole or the upstream region of a metapyrocatechase structural gene.

16. A DNA fragment according to claim 15 wherein the upstream region of said metapyrocatechase structural gene is the gene coding for about 35-200 amino acids at the N-end of the metapyrocatechase.

17. A plasmid containing a gene coding for a fish calcitonin derivative represented by the following amino acid sequence (I):

Cys Ser Asn Leu Ser Thr Cys Val Leu Gly
Lys Leu Ser Gln Glu Leu His Lys Leu Gln
Thr Tyr Pro Arg Thr X Y Gly Z Gly
Thr Pro Gly

wherein X represents Asn or Asp, Y represents Thr or Val, and Z represents Ser or Ala.

18. A plasmid according to claim 17 containing a gene coding for a salmon calcitonin derivative represented by the following amino acid sequence (II):

Cys Ser Asn Leu Ser Thr Cys Val Leu Gly
Lys Leu Ser Gln Glu Leu His Lys Leu Gln
Thr Tyr Pro Arg Thr Asn Thr Gly Ser Gly
Thr Pro Gly.

19. A plasmid according to claim 18 which is plasmid pSCT2.

20. A plasmid according to claim 18 containing an E. coli or phage promoter, a metapyrocatechase gene or a portion thereof, a gene coding for said salmon calcitonin derivative, and a terminator in that order.

21. A plasmid according to claim 20 wherein said promoter is a lacUV5 promoter, $P_L$ promoter or tac promoter.

22. A plasmid according to claim 20 wherein the terminator is a $\lambda t_{L1}$ terminator or tap a terminator.

23. A plasmid according to claim 20 which is plasmid pLMC2, plasmid pPMC2, plasmid pTMC2, plasmid pTMC22 or plasmid pTMC32.

24. A plasmid according to claim 17 containing a gene coding for the eel calcitonin derivative of the following amino acid sequence (III):

Cys Ser Asn Leu Ser Thr Cys Val Leu Gly
Lys Leu Ser Gln Glu Leu His Lys Leu Gln
Thr Tyr Pro Arg Thr Asp Val Gly Ala Gly
Thr Pro Gly

25. A plasmid according to claim 24 containing an E. coli promoter, a metapyrocatechase gene or a portion thereof, and a gene coding for said eel calcitonin derivative in that order.

26. A plasmid according to claim 25 wherein said promoter is tac promoter.

27. A plasmid according to claim 25 which is plasmid pCTM4.

28. E. coli transformed with plasmid containing a gene coding for a fish calcitonin derivative represented by the following amino acid sequence (I):

Cys Ser Asn Leu Ser Thr Cys Val Leu Gly

Lys Leu Ser Gln Glu Leu His Lys Leu Gln
Thr Tyr Pro Arg Thr X Y Gly Z Gly
Thr Pro Gly

wherein X represents Asn or Asp, Y represents Thr or Val, and Z represents Ser or Ala.

29. E. coli according to claim 28 which is Escherichia coli RR1/pSCT2 (FERM P-7775; FERM BP-866).

30. E. coli according to claim 28 whose host strain of E. coli is a strain RB791, strain HB101, strain JM103, strain C600, strain RR1, strain SM32, or strain W3110.

31. E. coli according to claim 28 which is Escherichia coli JM103/pLMC2 (FERM P-8220; FERM BP-867), Escherichia coli RR1/pPMC2 (FERM P-8222; FERM BP-868), Escherichia coli JM103/pTMC2 (FERM P-8223, FERM BP-869), Escherichia coli JM103/pTMC22, or Escherichia coli JM103/pTMC32 (FERM P8221).

32. A E. coli according to claim 28 which is Escherichia coli JM103/pCTM4 (FERM P-8239; FERM BP-870).

33. A process for production of a gene coding for a fish calcitonin derivative represented by the following amino acid sequence (I):

Cys Ser Asn Leu Ser Thr Cys Val Leu Gly
Lys Leu Ser Gln Glu Leu His Lys Leu Gln
Thr Tyr Pro Arg Thr X Y Gly Z Gly
Thr Pro Gly

wherein X represents Asn or Asp, Y represents Thr or Val, and Z represents Ser or Ala, said process comprising the steps of:

(1) synthesizing a plurality of oligonucleotides constituting the base sequence portion of said gene,

(2) constructing a plurality of subblocks by connecting said oligonucleotides, and

(3) connecting said subblocks.

34. A process according to claim 33 wherein the fish calcitonin derivative is a salmon calcitonin derivative.

35. A process for production of a gene coding for an eel calcitonin derivative characterized in that the codons coding for Asn at 26 position counting from Cys at the N-end of the salmon calcitonin derivative, Thr at 27 position, and Ser at 29 position in the gene coding for the salmon calcitonin derivative are replaced respectively by the codons coding for Asp, Val, and Ala.

36. A process according to claim 35 wherein said replacement is effected by the site directed mutagenesis using a obligonucleotide primer.

37. A fish calcitonin derivative represented by the following amino acid sequence (I):

Cys Ser Asn Leu Ser Thr Cys Val Leu Gly
Lys Leu Ser Gln Glu Leu His Lys Leu Gln
Thr Tyr Pro Arg Thr X Y Gly Z Gly
Thr Pro Gly,

wherein X represents Asn or Asp, Y represents Thr or Val, and Z represents Ser or Ala.

38. A fish calcitonin derivative according to claim 37 which is a salmon calcitonin derivative represented by the following amino acid sequence (II):

Cys Ser Asn Leu Ser Thr Cys Val Leu Gly
Lys Leu Ser Gln Glu Leu His Lys Leu Gln
Thr Tyr Pro Arg Thr Asn Thr Gly Ser Gly
Thr Pro Gly.

39. A process for preparation of a fish calcitonin derivative represented by the following amino acid sequence (I):

Cys Ser Asn Leu Ser Thr Cys Val Leu Gly
Lys Leu Ser Gln Glu Leu His Lys Leu Gln
Thr Tyr Pro Arg Thr X Y Gly Z Gly
Thr Pro Gly,

wherein X represents Asn or Asp, Y represents Thr or Val, and Z represents Ser or Ala, said process characterized by the steps of: culturing *E. coli* transformed by plasmid which contains a gene coding for said fish calcitonin derivative and a gene coding for another protein and is capable of expressing these genes in E. coli to produce a fused protein consisting of said fish calcitonin derivative and said another protein, recovering said fused protein, cutting the recovered fused protein to liberate said fish calcitonin derivative, and recovering the derivative.

40. A process according to calim 39 characterized by the steps of: culturing E. coli transformed by plasmid containing an E. coli or phage promoter, a metapyrocatechase gene or a portion thereof, a gene coding for the salmon calcitonin derivative consisting of the following amio acid sequence (II):

Cys Ser Asn Leu Ser Thr Cys Val Leu Gly
Lys Leu Ser Gln Glu Leu His Lys Leu Gln
Thr Tyr Pro Arg Thr Asn Thr Gly Ser Gly
Thr Pro Gly,

and a terminater in that order to produce a fused protein consisting of the metapyrocatechase or a portion thereof and the salmon calcitonin derivative in the cells, recovering said fused protein from said cells, cutting the recovered fused protein to liberate said salmon calcitonin derivative, and recovering the derivative.

41. A process according to claim 39 characterized by the steps of: culturing E. coli transformed by plasmid containing an E. coli promoter, a metapyrocatechase gene or a portion thereof, and a gene coding for an eel calcitonin derivative consisting of the following amino acid sequence (III).

Cys Ser Asn Leu Ser Thr Cys Val Leu Gly
Lys Leu Ser Gln Glu Leu His Lys Leu Gln
Thr Tyr Pro Arg Thr Asp Val Gly Ala Gly
Thr Pro Gly,

to produce a fused protein consisting of the metapyrocatechase or a portion thereof and the eel calcitonin derivative in the cells, recovering said fused protein from said cells, cutting the recovered fused protein to liberate said eel calcitonin derivative, and recovering this eel calcitonin derivative.

42. A fish calcitonin derivative according to claim 37 which is an eel calcitonin derivative represented by the following amino acid sequence (III):

Cys Ser Asn Leu Ser Thr Cys Val Leu Gly
Lys Leu Ser Gln Glu Leu His Lys Leu Gln
Thr Tyr Pro Arg Thr Asp Val Gly Ala Gly
Thr Pro Gly.

```
          1   2   3   4   5   6   7   8   9   10  11  12  13  14  15  16  17  18  19
      Met Cys Ser Asn Leu Ser Thr Cys Val Leu Gly Lys Leu Ser Gln Glu Leu His Lys Leu
          U-11 (19)           U-2 (17)            U-31 (15)           U-41 (15)
     GTT AAC ATG TGC TCC AAT CTC TCT ACT TGC GTT CTG GGG AAG TTG AGT CAG GAA TTA CAT AAG CTG
ACGT CAA TTG TAC ACG AGG TTA GAG AGA TGA ACG CAA GAC CCC TTC AAC TCA GTC CTT AAT G
     L-11 (15)           L-2 (17)            L-31 (15)           L-41 (15)

17  18  19  20  21  22  23  24  25  26  27  28  29  30  31  32  33
His Lys Leu Gln Thr Tyr Pro Arg Thr Asn Thr Gly Ser Gly Thr Pro Gly stp stp
               U-5 (15)            U-6 (17)            U-7 (18)
            CAA ACT TAC CCG CGT ACC AAC ACT GGT TCT GGT ACA CCT GGT TAA TAG AT
 TA TTC GAC GTT TGA ATG GGC GCA TGG TTG TGA CCA AGA CCA TGT GGA CCA ATT ATC TAG C
         L-5 (16)            L-6 (17)            L-7 (14)         L-8 (13)

                                            U-7' (15)
                                      T ACA CCT TAA TAG AT
                  Without Gly (33) codon
                                            ATT ATC TAG C
                                             L-8' (10)
```

Fig. 1

```
     1   2   3   4   5   6   7   8   9   10  11  12  13  14  15  16  17  18  19
 Met Cys Ser Asn Leu Ser Thr Cys Val Leu Gly Lys Leu Ser Gln Glu Leu His Lys Leu
     U-1 (16)                U-2 (17)                U-31 (15)       U-41 (15)
AAC ATG TGC TCC AAT CTC TCT ACT TGC GTT CTG GGG AAG TTG AGT CAG GAA TTA CAT AAG CTG

TTG TAC ACG AGG TTA GAG AGA TGA ACG CAA GAC CCC TTC AAC TCA GTC CTT AAT G
 L-1 (8)            L-2 (17)              L-31 (15)           L-41 (15)


 17  18  19  20  21  22  23  24  25  26  27  28  29  30  31  32  33
 His Lys Leu Gln Thr Tyr Pro Arg Thr Asn Thr Gly Ser Gly Thr Pro Gly stp stp
                 U-5 (15)                U-6 (17)                U-7 (18)
             CAA ACT TAC CCG CGT ACC AAC ACT GGT TCT GGT ACA CCT GGT TAA TAG AT

 TA TTC GAC GTT TGA ATG GGC GCA TGG TTG TGA CCA AGA CCA TGT GGA CCA ATT ATC TAG C
         L-5 (16)               L-6 (17)            L-7 (14)           L-8 (13)


                                                      U-7' (15)
                                               T ACA CCT TAA TAG AT
                            Without Gly (33) codon
                                                  ATT ATC TAG C
                                                    L-8' (10)
```

Fig. 2

Fig. 3

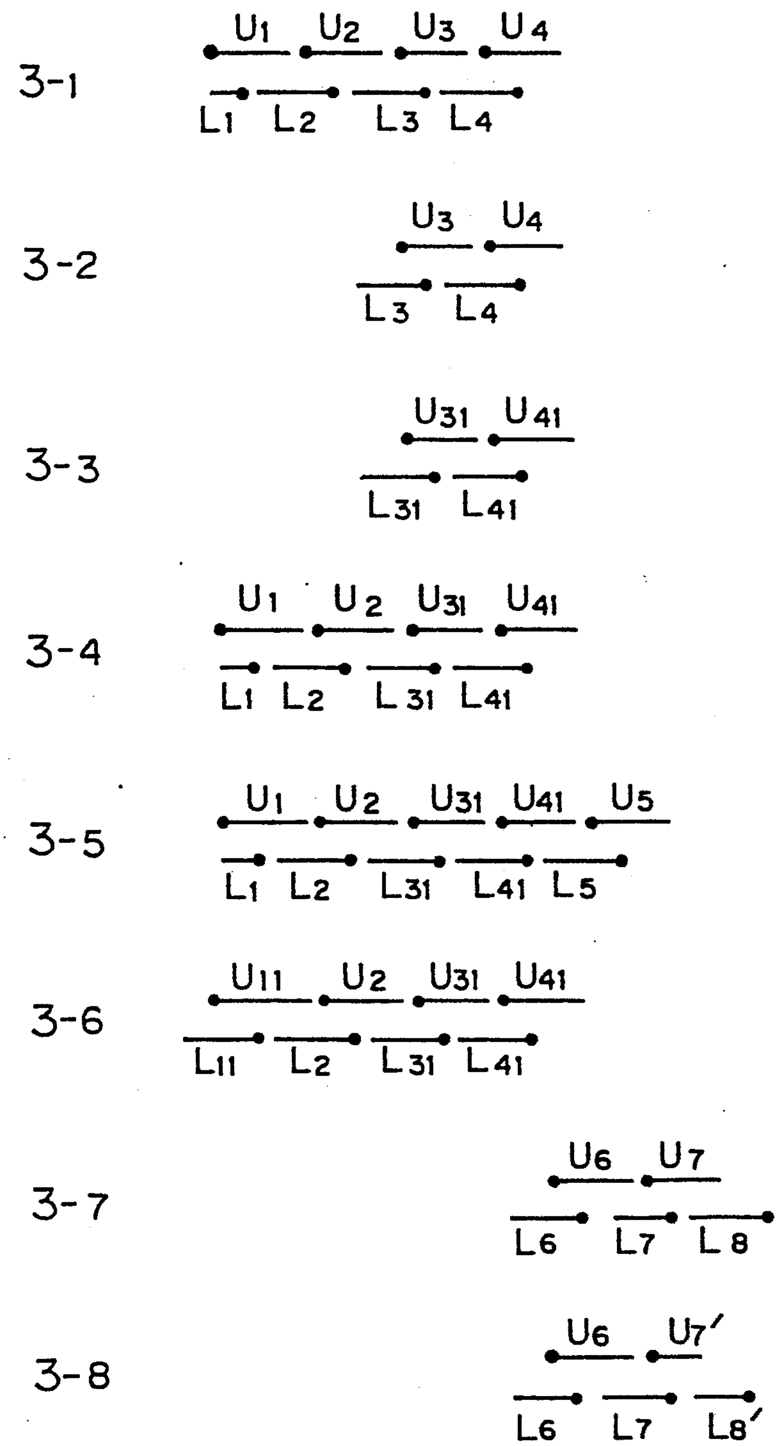
3-1
U1 U2 U3 U4
L1 L2 L3 L4
3-2
U3 U4
L3 L4
3-3
U31 U41
L31 L41
3-4
U1 U2 U31 U41
L1 L2 L31 L41
3-5
U1 U2 U31 U41 U5
L1 L2 L31 L41 L5
3-6
U11 U2 U31 U41
L11 L2 L31 L41
3-7
U6 U7
L6 L7 L8
3-8
U6 U7′
L6 L7 L8′

Fig. 4

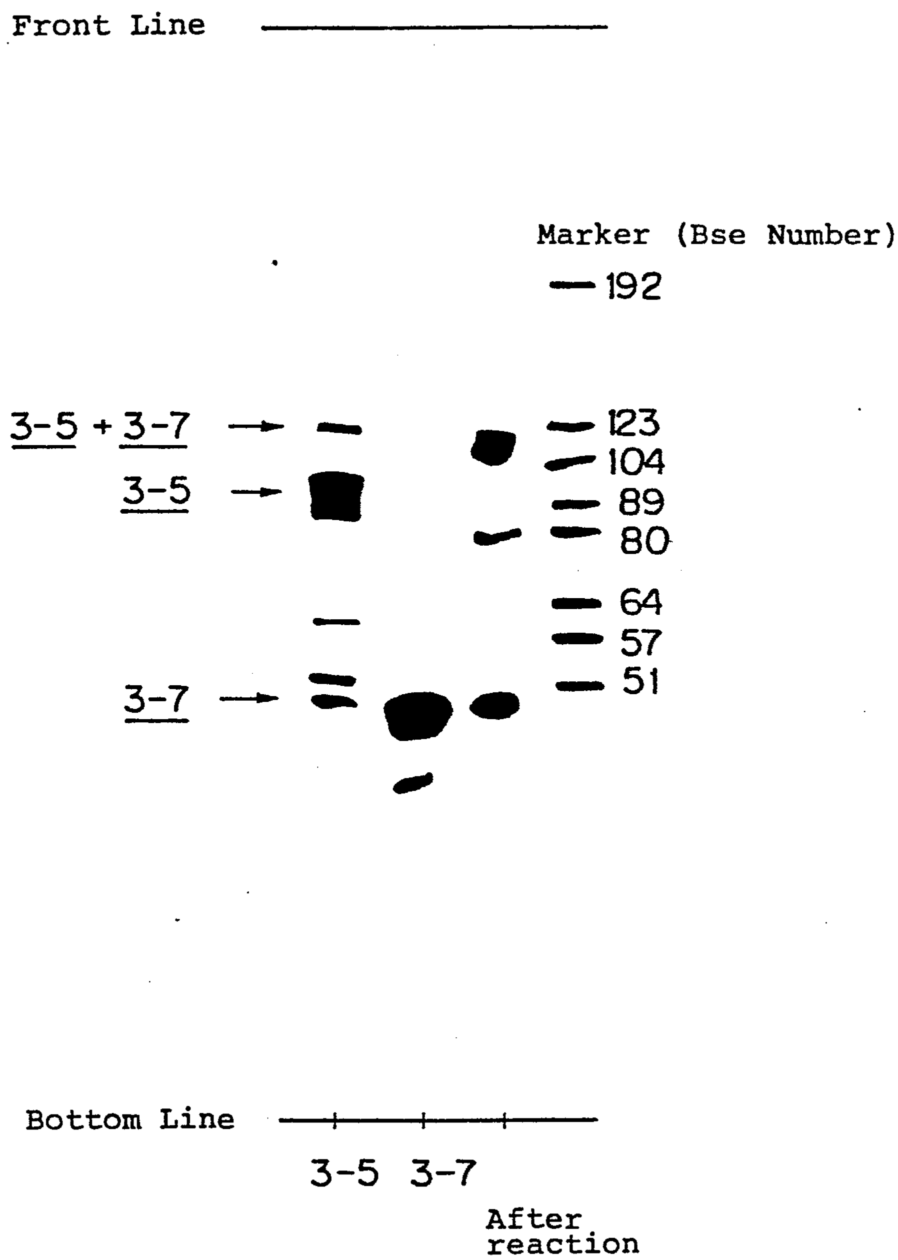
Front Line
Marker (Bse Number)
192
3-5 + 3-7
3-5
123
104
89
80
64
57
51
3-7
Bottom Line
3-5
3-7
After reaction

*Fig. 5*

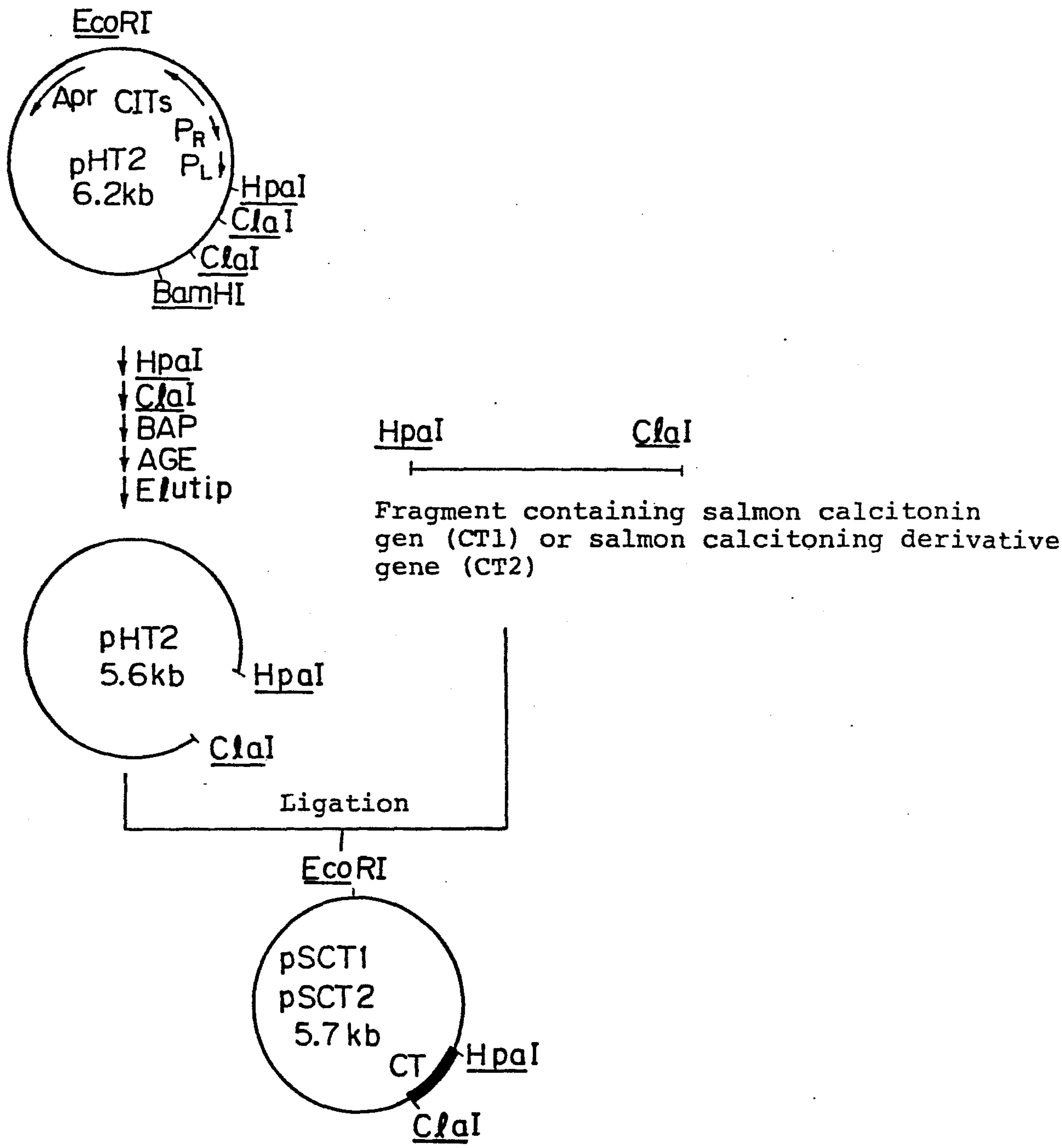
EcoRI
Apr
CITs
$P_R$
$P_L$
pHT2
6.2kb
HpaI
ClaI
ClaI
BamHI
HpaI
ClaI
BAP
AGE
Elutip
HpaI
ClaI
Fragment containing salmon calcitonin gen (CT1) or salmon calcitoning derivative gene (CT2)
pHT2
5.6kb
HpaI
ClaI
Ligation
EcoRI
pSCT1
pSCT2
5.7 kb
CT
HpaI
ClaI

Fig. 6

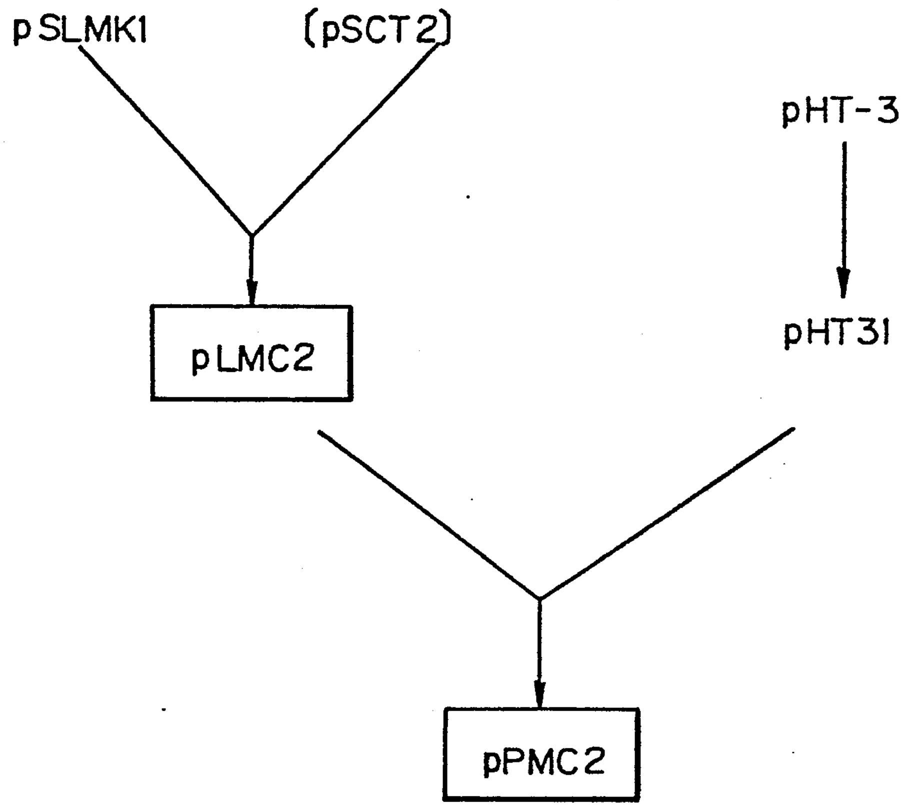

Fig. 7

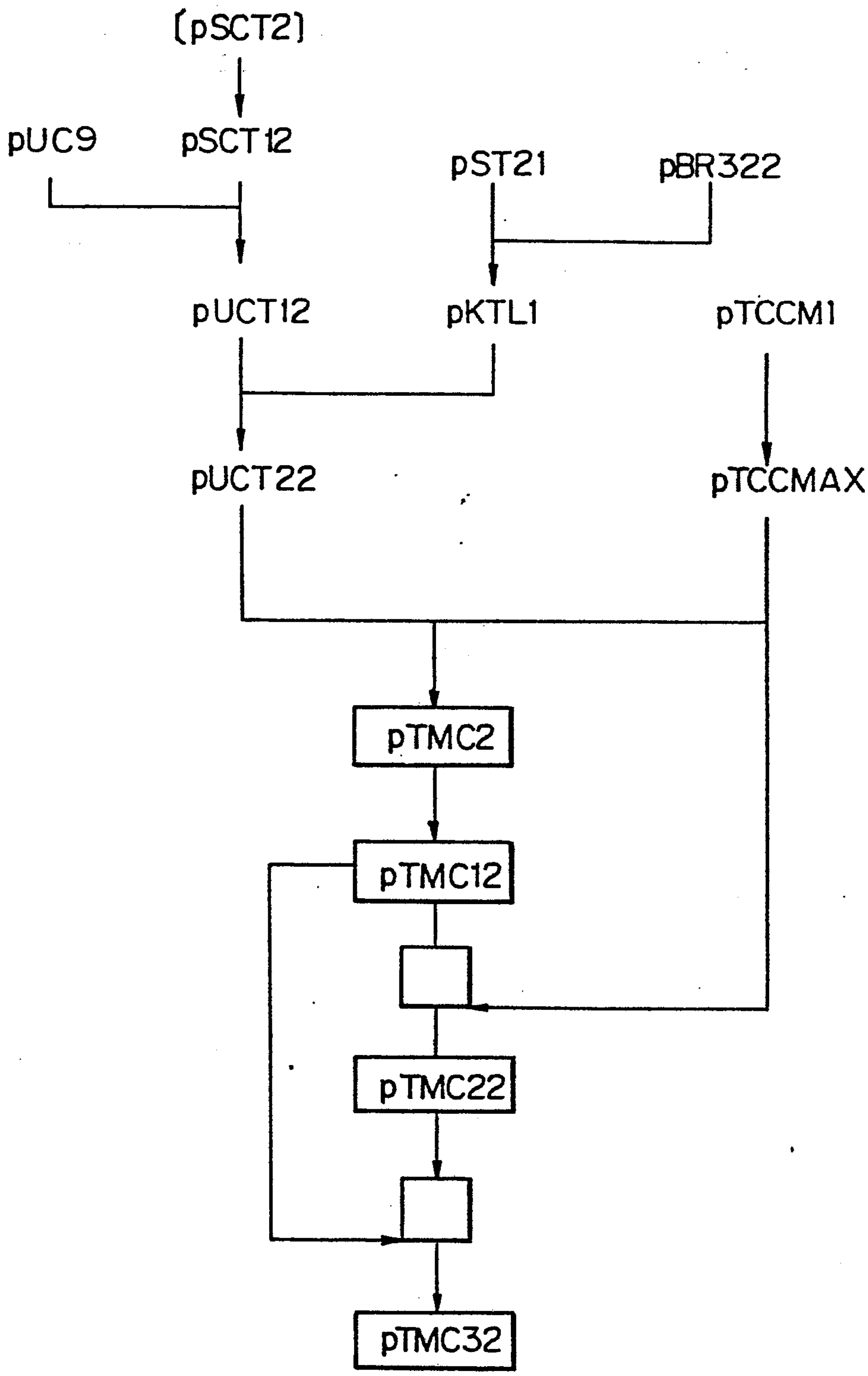
(pSCT2)
pUC9
pSCT12
pST21
pBR322
pUCT12
pKTL1
pTCCM1
pUCT22
pTCCMAX
pTMC2
pTMC12
pTMC22
pTMC32

# Fig. 8

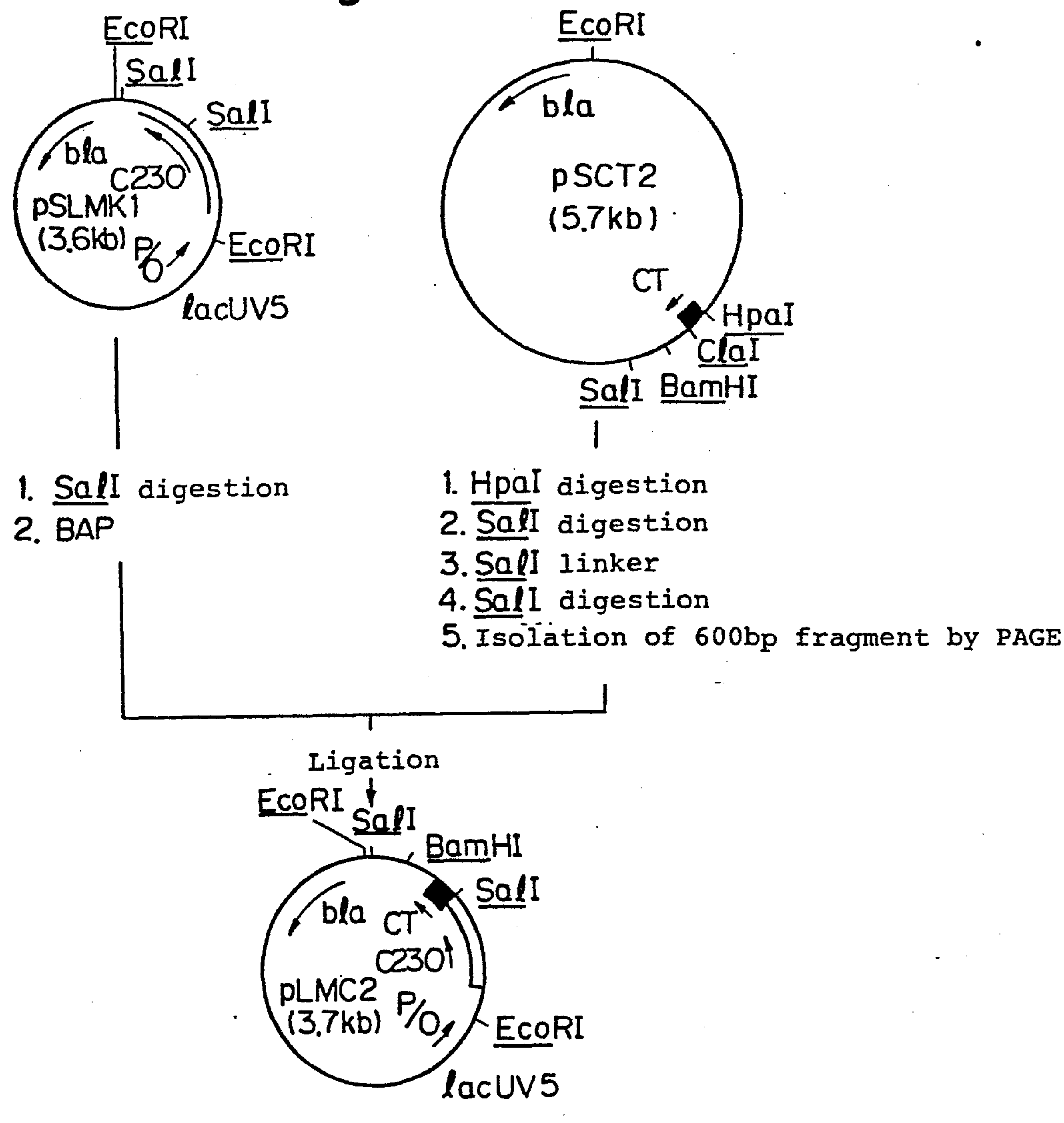


(A)

—— C230 —————— SalI —————— I—CT ——————→

G TTT CTC AGT CTG TCG ACC AAC ATG TGC TCC AAT CTC TCT ACT

C AAA GAG TCA GAC AGC TGG TTG TAC ACG AGG TTA GAG AGA TGA

(B)

Fig. 9

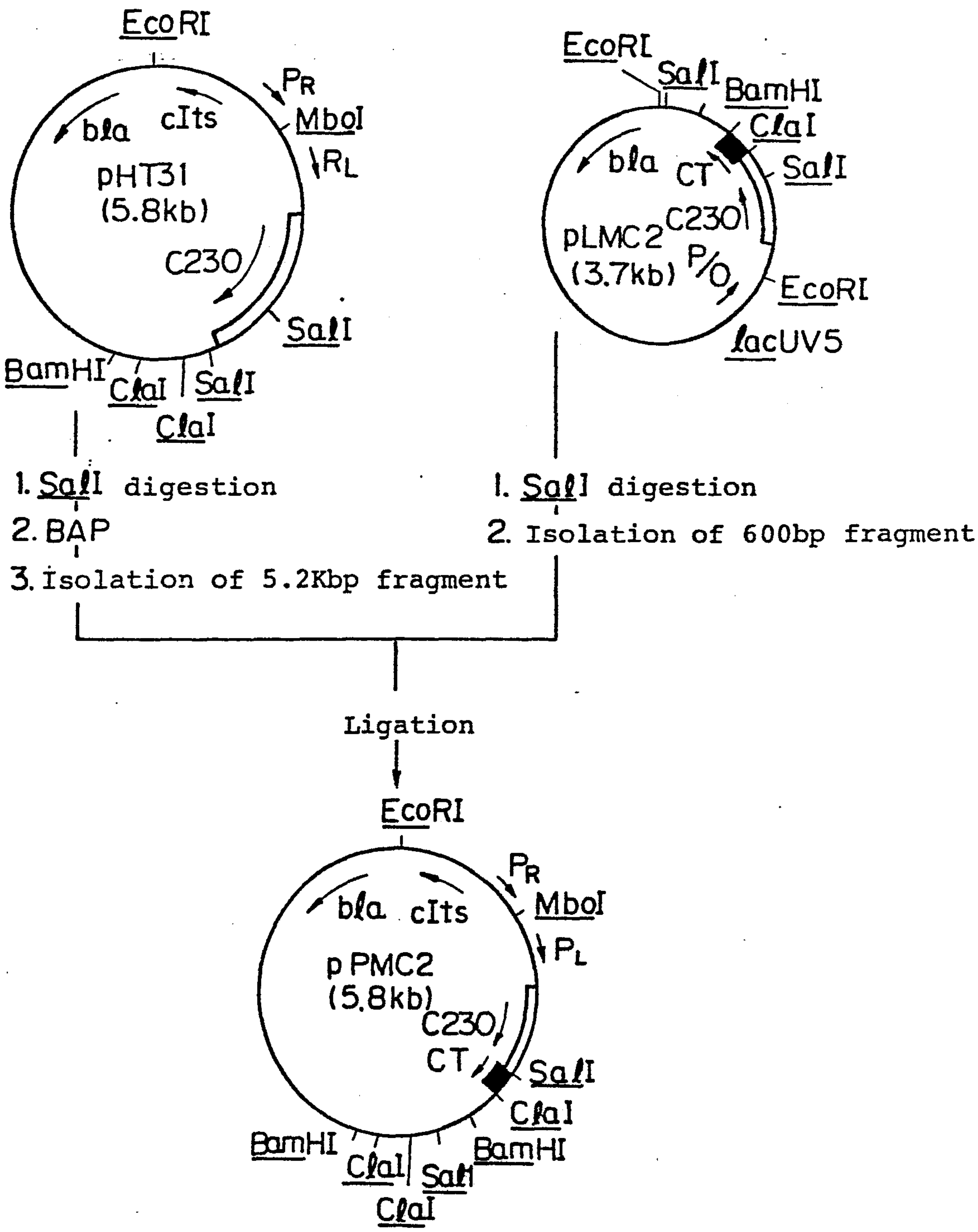
EcoRI
PR
MboI
RL
bla
cIts
pHT31
(5.8kb)
C230
SalI
BamHI
ClaI
SalI
ClaI
EcoRI
SalI
BamHI
ClaI
bla
CT
SalI
C230
pLMC2
(3.7kb)
P/O
EcoRI
lacUV5
1. SalI digestion
2. BAP
3. Isolation of 5.2Kbp fragment
1. SalI digestion
2. Isolation of 600bp fragment
Ligation
EcoRI
PR
MboI
PL
bla
cIts
p PMC2
(5.8kb)
C230
CT
SalI
ClaI
BamHI
BamHI
ClaI
SalI
ClaI

Fig. 10

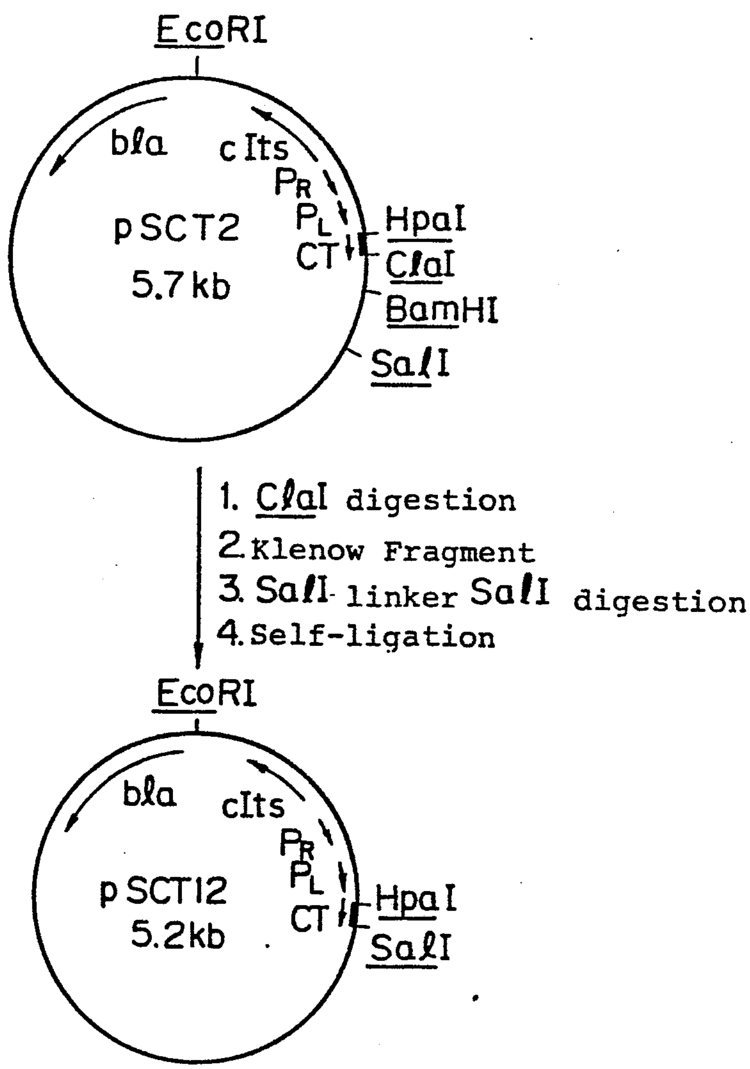
EcoRI
bla
c Its
PR
PL
CT
pSCT2
5.7 kb
HpaI
ClaI
BamHI
SalI
1. ClaI digestion
2. Klenow Fragment
3. SalI linker SalI digestion
4. Self-ligation
EcoRI
bla
cIts
PR
PL
CT
pSCT12
5.2kb
HpaI
SalI

# Fig. 11

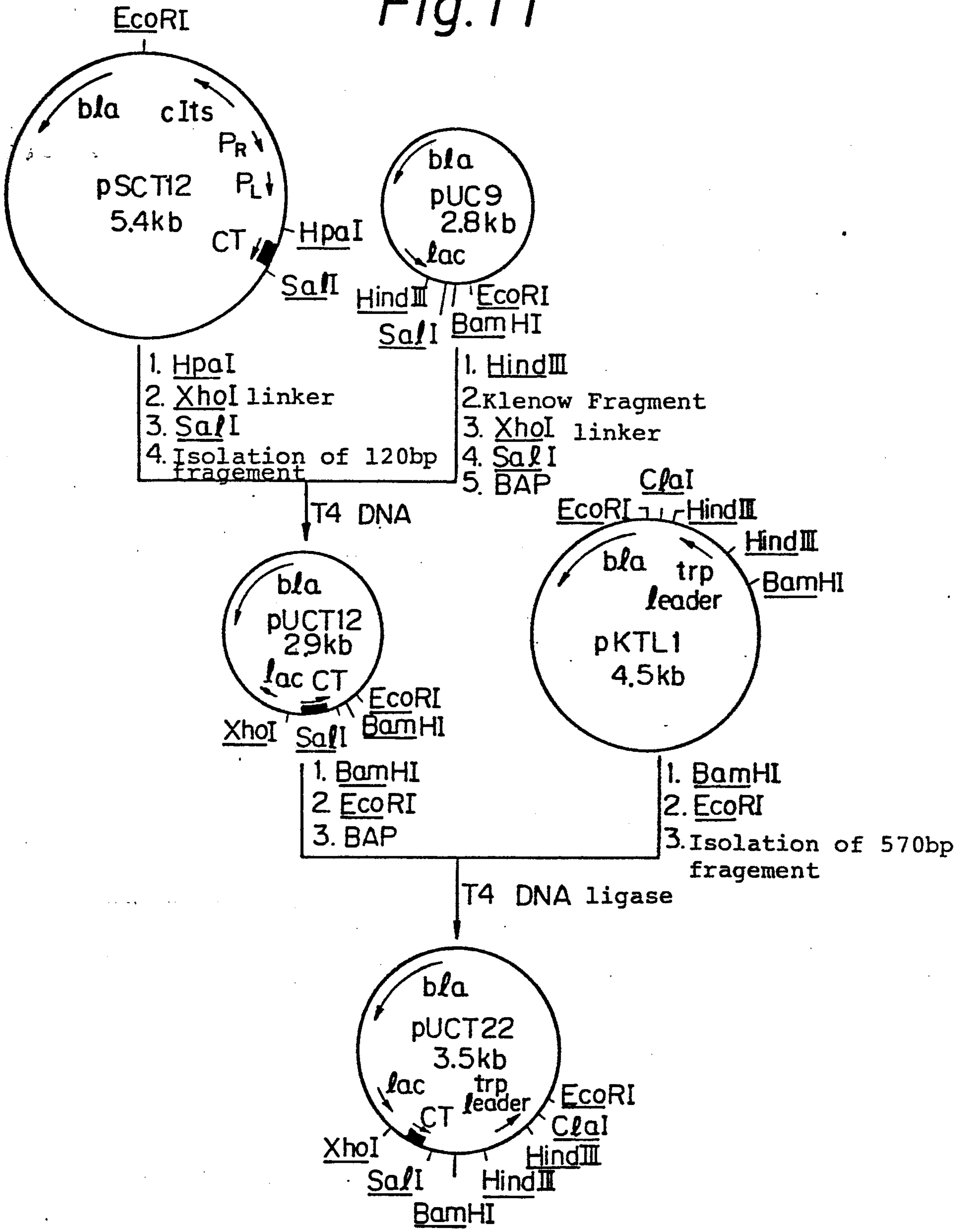
EcoRI
bla
cIts
PR
PL
pSCT12
5.4kb
CT
HpaI
SalI
bla
pUC9
2.8kb
lac
HindIII
EcoRI
SalI
BamHI
1. HpaI
2. XhoI linker
3. SalI
4. Isolation of 120bp fragement
1. HindIII
2. Klenow Fragment
3. XhoI linker
4. SalI
5. BAP
T4 DNA
ClaI
EcoRI
HindIII
HindIII
BamHI
bla
trp
leader
pKTL1
4.5kb
bla
pUCT12
2.9kb
lac
CT
EcoRI
BamHI
XhoI
SalI
1. BamHI
2. EcoRI
3. BAP
1. BamHI
2. EcoRI
3. Isolation of 570bp fragement
T4 DNA ligase
bla
pUCT22
3.5kb
lac
trp
leader
CT
EcoRI
ClaI
HindIII
HindIII
XhoI
SalI
BamHI

Fig.12

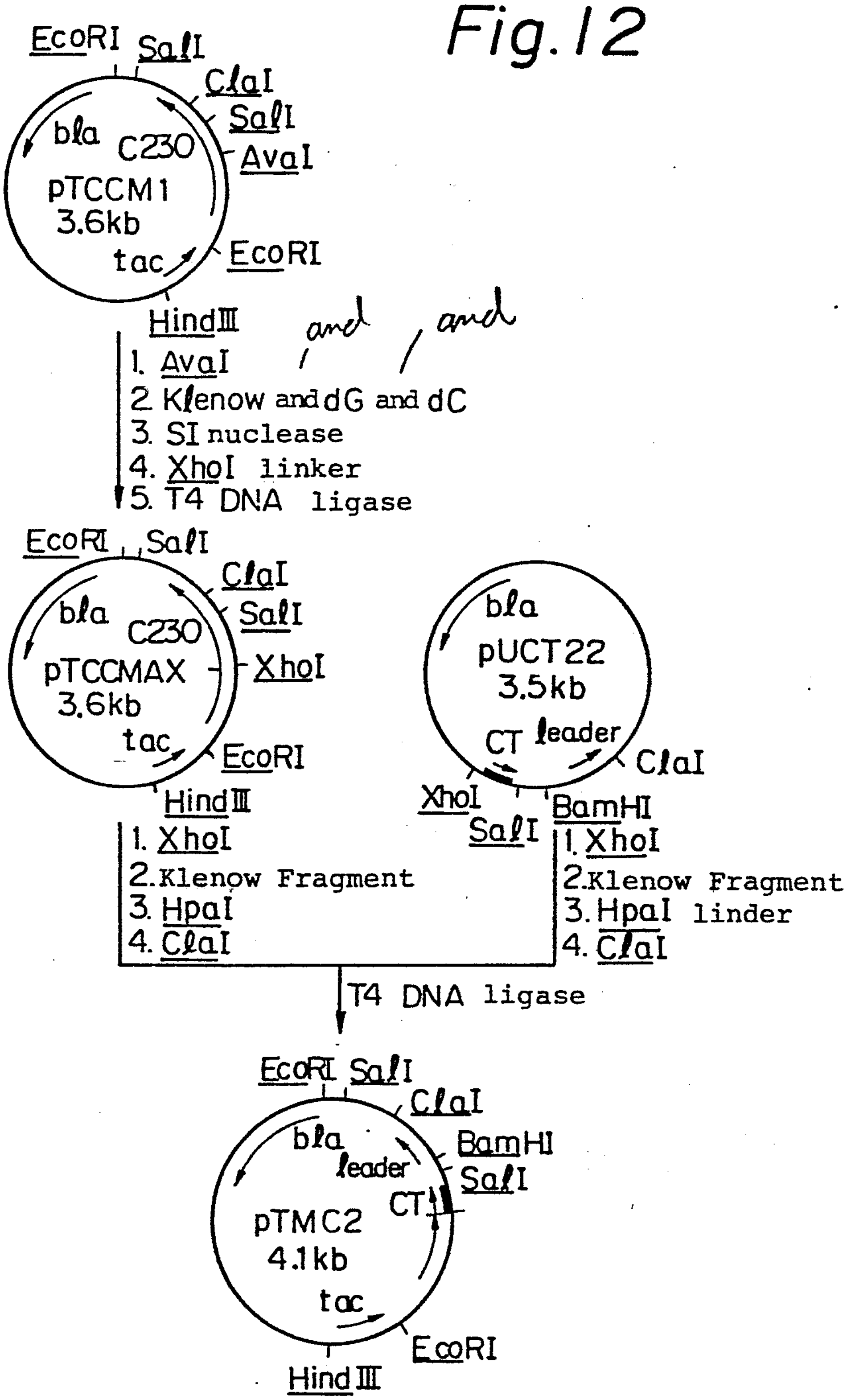
EcoRI
SalI
ClaI
SalI
AvaI
bla
C230
pTCCM1
3.6kb
tac
EcoRI
HindIII
1. AvaI
2. Klenow and dG and dC
3. SI nuclease
4. XhoI linker
5. T4 DNA ligase
and
and
EcoRI
SalI
ClaI
SalI
XhoI
bla
C230
pTCCMAX
3.6kb
tac
EcoRI
HindIII
bla
pUCT22
3.5kb
CT
leader
ClaI
XhoI
SalI
BamHI
1. XhoI
2. Klenow Fragment
3. HpaI
4. ClaI
1. XhoI
2. Klenow Fragment
3. HpaI linder
4. ClaI
T4 DNA ligase
EcoRI
SalI
ClaI
BamHI
SalI
bla
leader
CT
pTMC2
4.1kb
tac
EcoRI
HindIII

(A)

Fig. 12

132 135 140 143

-AsnAspValAsnProProArgAsnSerArgAsnMetCys-

-AATGACTGCAATCCCCCTCGAAACTCGAGGAACATGTGC--
-TTACTGCAGTTAGGGGGAGCTTTGAGCTCCTTGTACACG--

——— C230 → - - - - - XhoI - - - - + ——→ CT

(B)

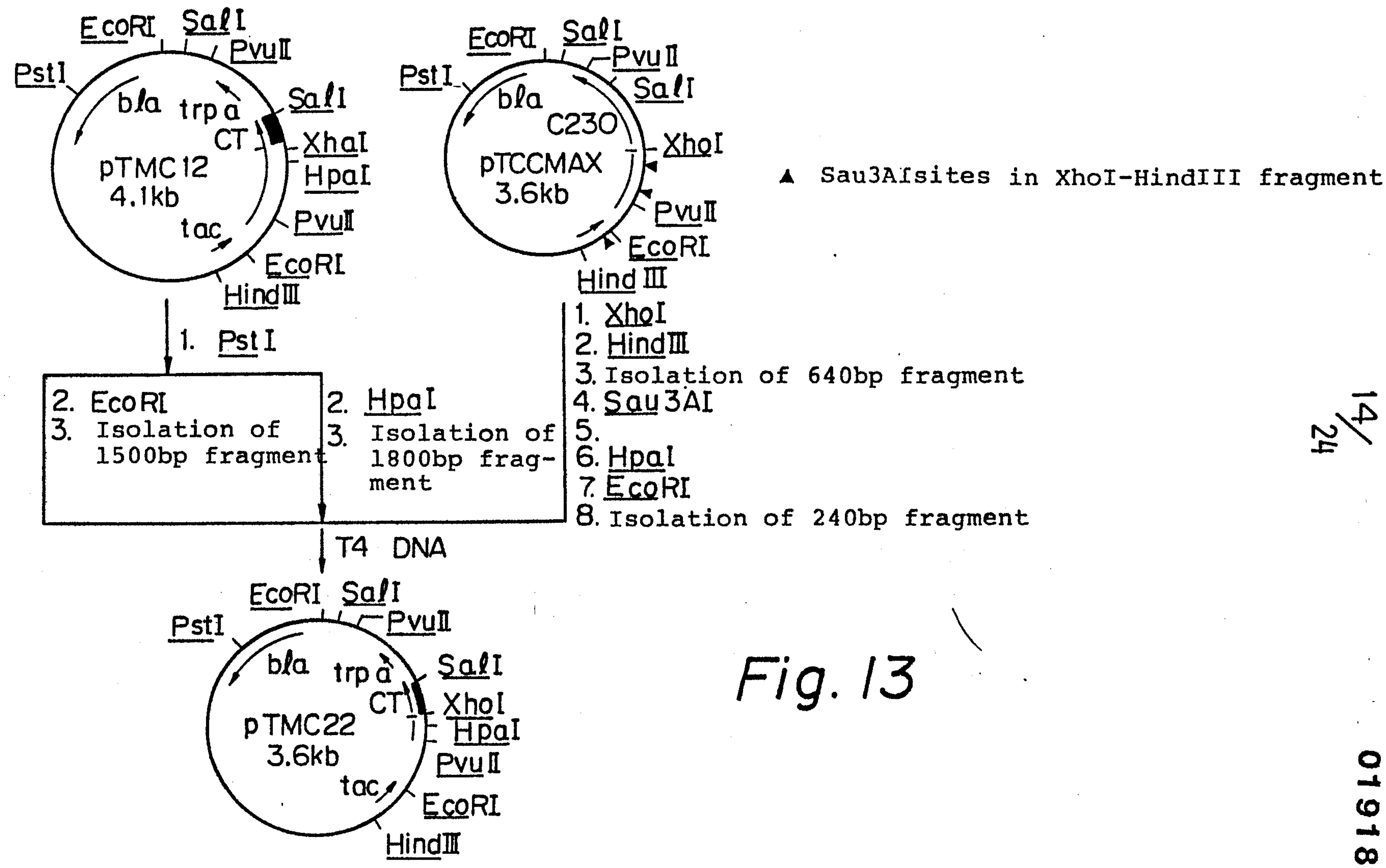
EcoRI
SalI
PvuII
PstI
bla
trp a
SalI
CT
XhaI
HpaI
pTMC12
4.1kb
tac
PvuII
EcoRI
HindIII
1. PstI
2. EcoRI
3. Isolation of 1500bp fragment
2. HpaI
3. Isolation of 1800bp fragment
EcoRI
SalI
PvuII
PstI
bla
SalI
C230
XhoI
pTCCMAX
3.6kb
PvuII
EcoRI
HindIII
1. XhoI
2. HindIII
3. Isolation of 640bp fragment
4. Sau3AI
5.
6. HpaI
7. EcoRI
8. Isolation of 240bp fragment
T4 DNA
EcoRI
SalI
PvuII
PstI
bla
trp a
SalI
CT
XhoI
HpaI
pTMC22
3.6kb
PvuII
tac
EcoRI
HindIII
▲ Sau3AIsites in XhoI-HindIII fragment

Fig. 13

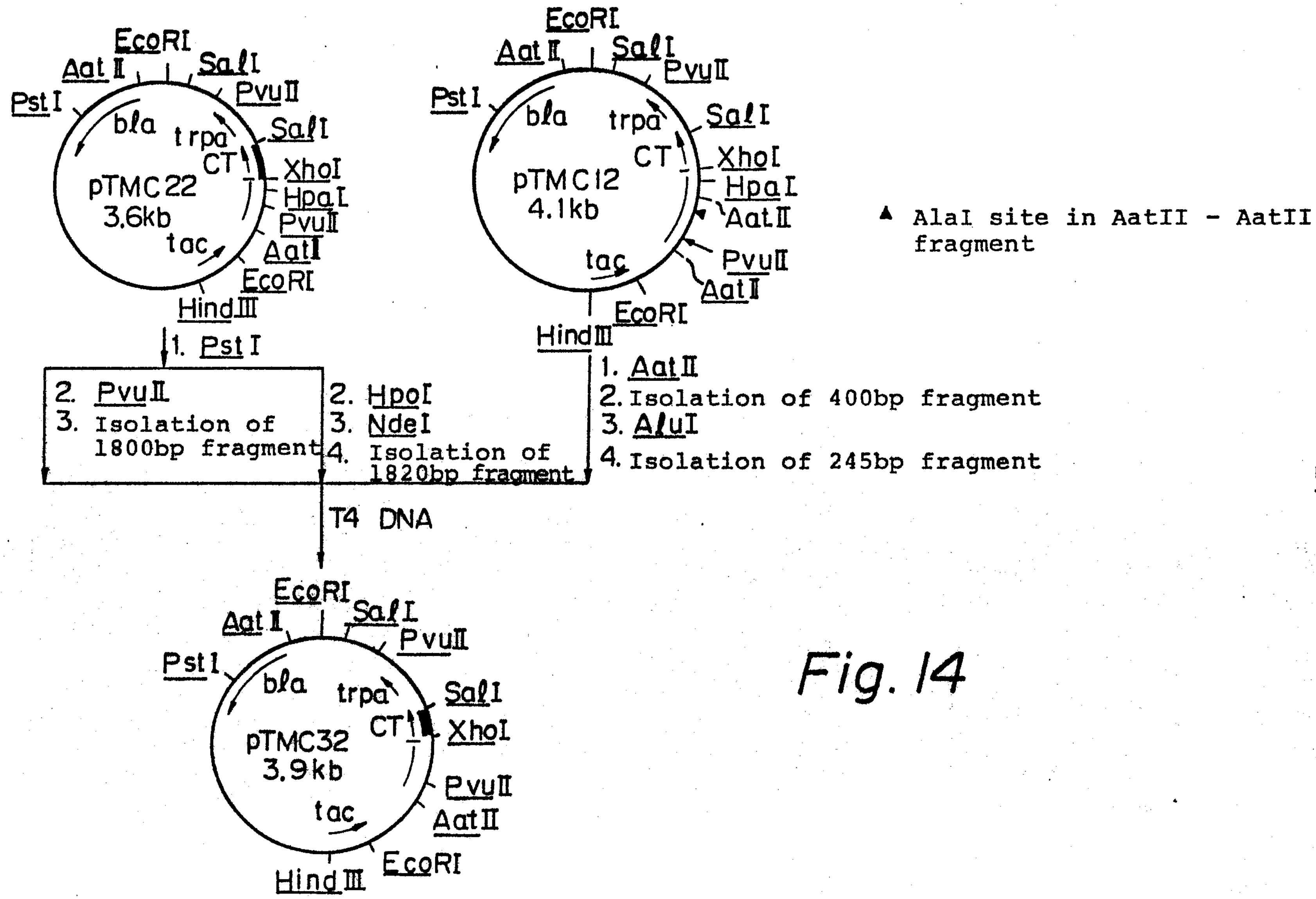

pTMC22
3.6kb
PstI
AatII
EcoRI
SalI
PvuII
SalI
XhoI
HpaI
PvuII
AatII
EcoRI
HindIII
bla
trpa
CT
tac
1. PstI
2. PvuII
3. Isolation of 1800bp fragment
2. HpoI
3. NdeI
4. Isolation of 1820bp fragment
pTMC12
4.1kb
PstI
AatII
EcoRI
SalI
PvuII
SalI
XhoI
HpaI
AatII
PvuII
AatII
EcoRI
HindIII
bla
trpa
CT
tac
1. AatII
2. Isolation of 400bp fragment
3. AluI
4. Isolation of 245bp fragment
T4 DNA
pTMC32
3.9kb
PstI
AatII
EcoRI
SalI
PvuII
SalI
XhoI
PvuII
AatII
EcoRI
HindIII
bla
trpa
CT
tac
▲ AlaI site in AatII - AatII fragment


Fig. 14

Fig. 15

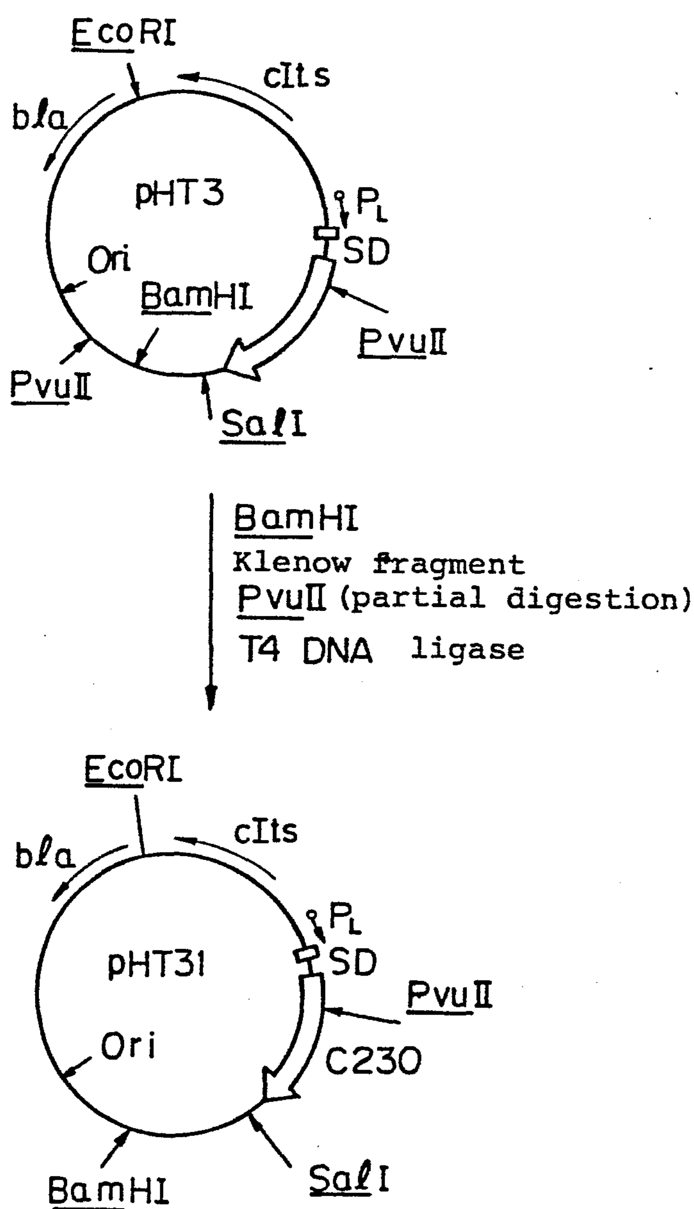
EcoRI
cIts
bla
pHT3
PL
SD
Ori
BamHI
PvuII
PvuII
SalI
BamHI
Klenow fragment
PvuII (partial digestion)
T4 DNA ligase
EcoRI
cIts
bla
PL
pHT31
SD
PvuII
Ori
C230
BamHI
SalI

Fig. 16

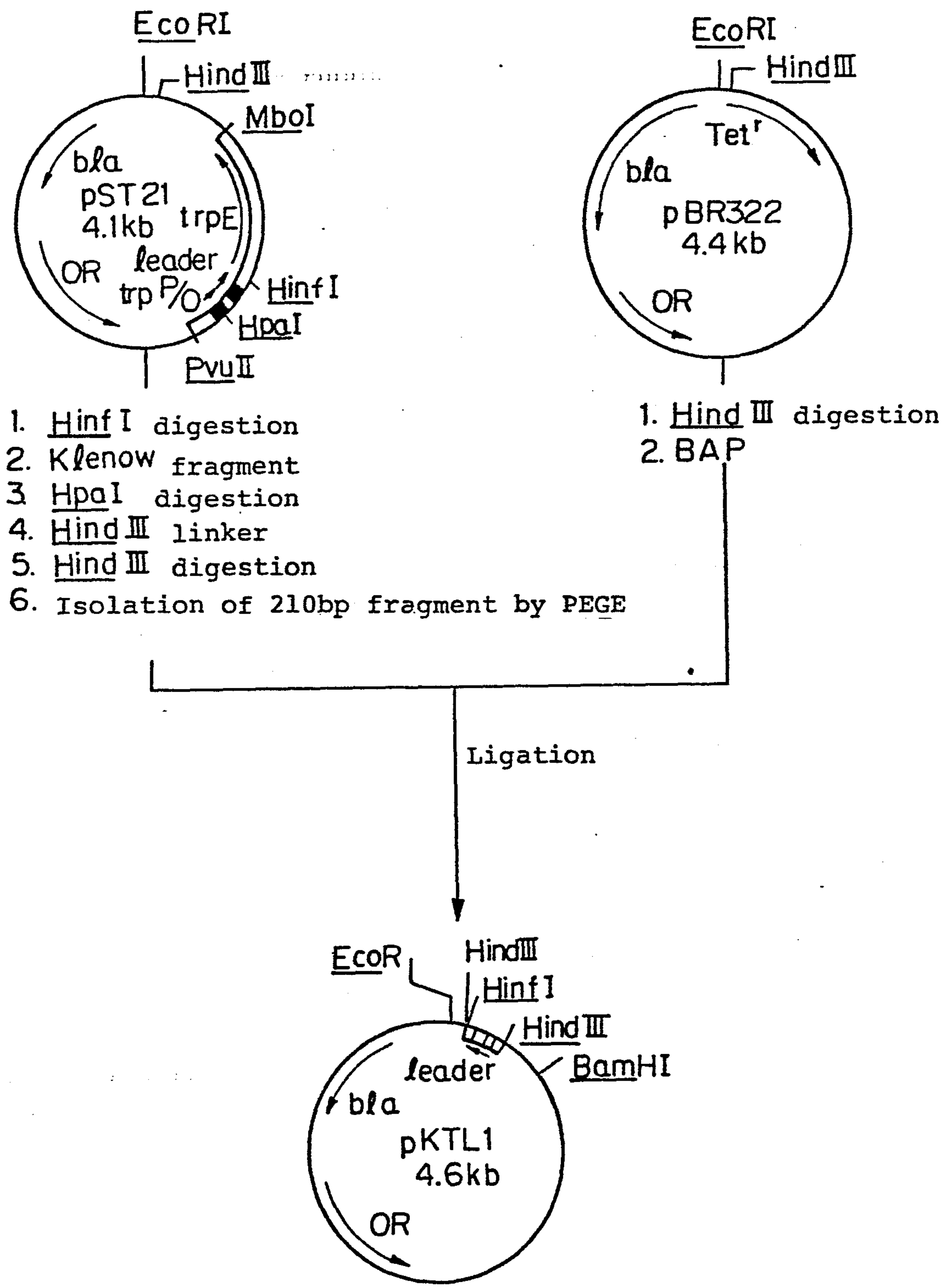
EcoRI
HindIII
MboI
bla
pST21
4.1kb
trpE
leader
OR
trp P/O
HinfI
HpaI
PvuII
1. HinfI digestion
2. Klenow fragment
3. HpaI digestion
4. HindIII linker
5. HindIII digestion
6. Isolation of 210bp fragment by PEGE
EcoRI
HindIII
Tet^r
bla
pBR322
4.4kb
OR
1. HindIII digestion
2. BAP
Ligation
EcoR
HindIII
HinfI
HindIII
BamHI
leader
bla
pKTL1
4.6kb
OR

# Fig. 17

| | | | | | | | | | | | | | | | | | | | | | |
|---|---|---|---|---|---|---|---|---|---|---|---|---|---|---|---|---|---|---|---|---|---|
| Salmon Calcitonin Derivative | Anticode Chain ... | ACC | AGG | TGT | ACC | AGA | ACC | AGT | GTT | GGT | ACG | CGG | GTA | AGT | ----- | GCA | CAT | GTT | GGG | AAT | T |
| | Code Chain.. | TGG | TCC | ACA | TGG | TCT | TGG | TCA | CAA | CCA | TGC | GCC | CAT | TCA | ----- | CGT | GTA | CAA | CCC | TTA | A |
| | Amino Acid.. Sequence | Gly | Pro | Thr | Gly | Ser | Gly | Thr | Asn | Thr | Arg | Pro | Tyr | Thr | ----- | Cys | Met | Asn | Pro | Ile | |
| | | 33 | 32 | 31 | 30 | 29 | 28 | 27 | 26 | 25 | 24 | 23 | 22 | 21 | | 1 | 0 | | | | |
| Eel Calcitonin Derivative | Anticode Chain ... | ACC | AGG | TGT | ACC | AGC | ACC | AAC | GTC | GGT | ACG | CGG | GTA | AGT | ----- | GCA | CAT | GTT | GGG | AAT | T |
| | Code Chain.. | TGG | TCC | ACA | TGG | TCG | TGG | TTG | CAG | CCA | TGC | GCC | CAT | TCA | ----- | CGT | GTA | CAA | CCC | TTA | A |
| | Amino Acid.. Sequence | Gly | Pro | Thr | Gly | Ala | Gly | Val | Asp | Thr | Arg | Pro | Tyr | Thr | ----- | Cys | Met | Asn | Pro | Ile | |
| | | 33 | 32 | 31 | 30 | 29 | 28 | 27 | 26 | 25 | 24 | 23 | 22 | 21 | | 1 | 0 | | | | |
| Primer Oligonucleotide | | | | | TGG | TCG | TGG | TTG | CAG | CCA | TGC | GC | | | | | | | | | |
| Probe Oligonucleotide | | | | | | TCG | TGG | TTG | CAG | CCA | | | | | | | | | | | |

EcoRI (at GGG | AAT T / TTA A, above each derivative)

(Ligation site)

Fig. 18

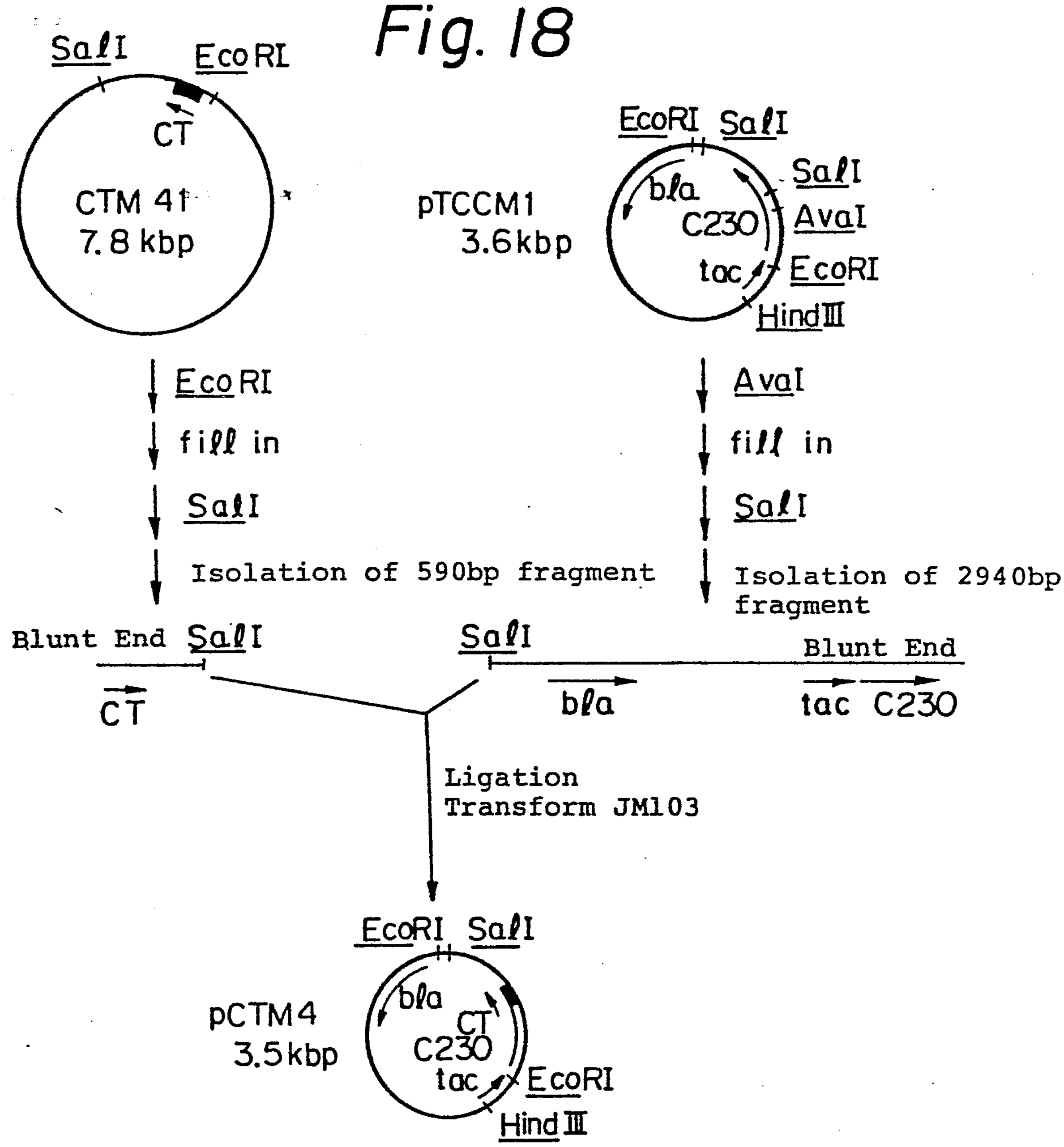


(A)

Asn Pro Glu Ile Pro Asn Met Cys

--- AAT C cc ga A ATT CCC AAC ATG TGC ---

---TTA G GG CT t taa GGG TTG TAC ACG---

135 136 | 0

Derived from pTCCM1 | Derived from CTM41

(B)

# Fig. 19

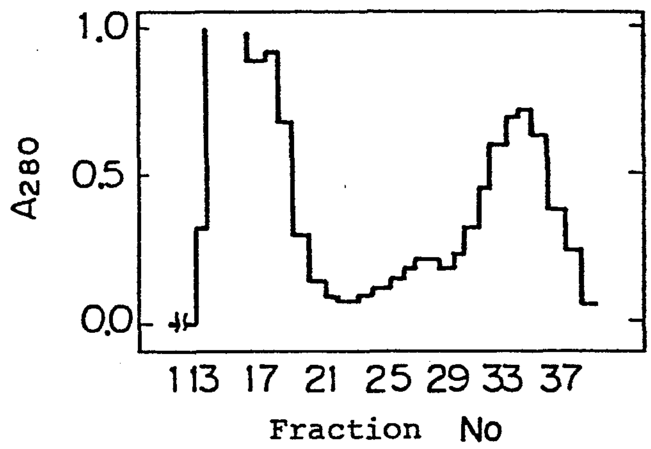

1.0
0.5
0.0
A280
1 13 17 21 25 29 33 37
Fraction No


(A)

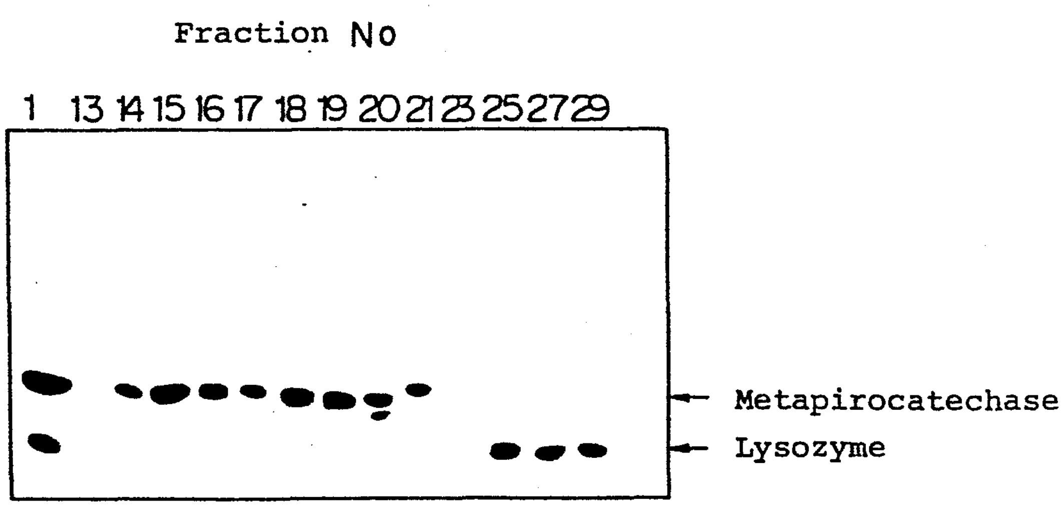

Fraction No
1 13 14 15 16 17 18 19 20 21 23 25 27 29
Metapirocatechase
Lysozyme


(B)

Fig. 20

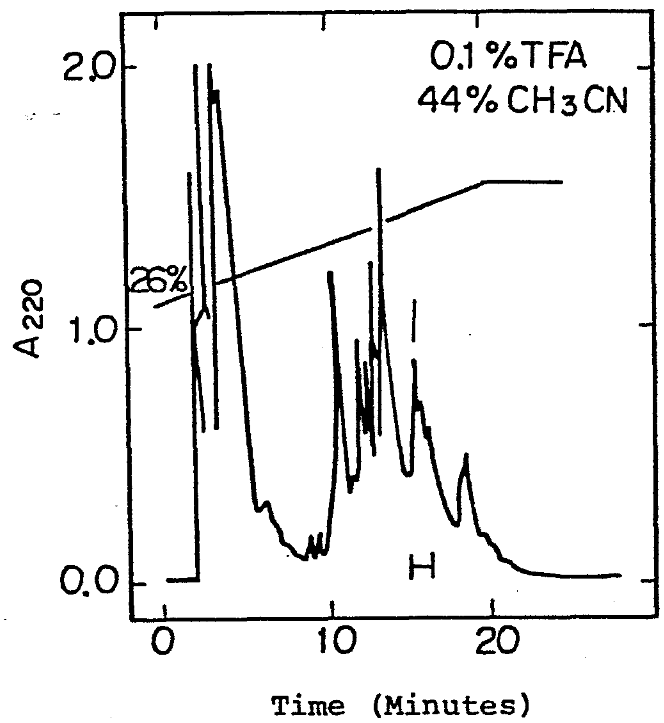

0.1%TFA
44% CH3CN
26%
H
2.0
1.0
0.0
A220
0
10
20
Time (Minutes)


(A)

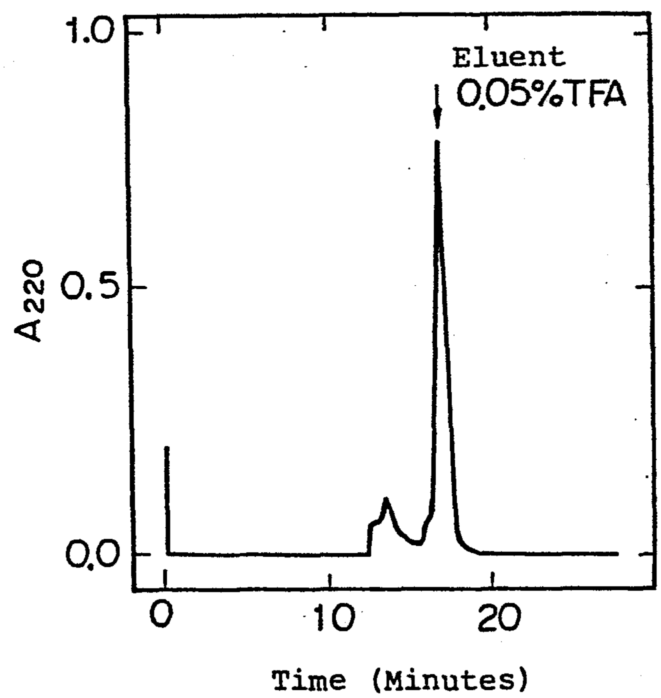

Eluent
0.05%TFA
1.0
0.5
0.0
A220
0
10
20
Time (Minutes)


(B)

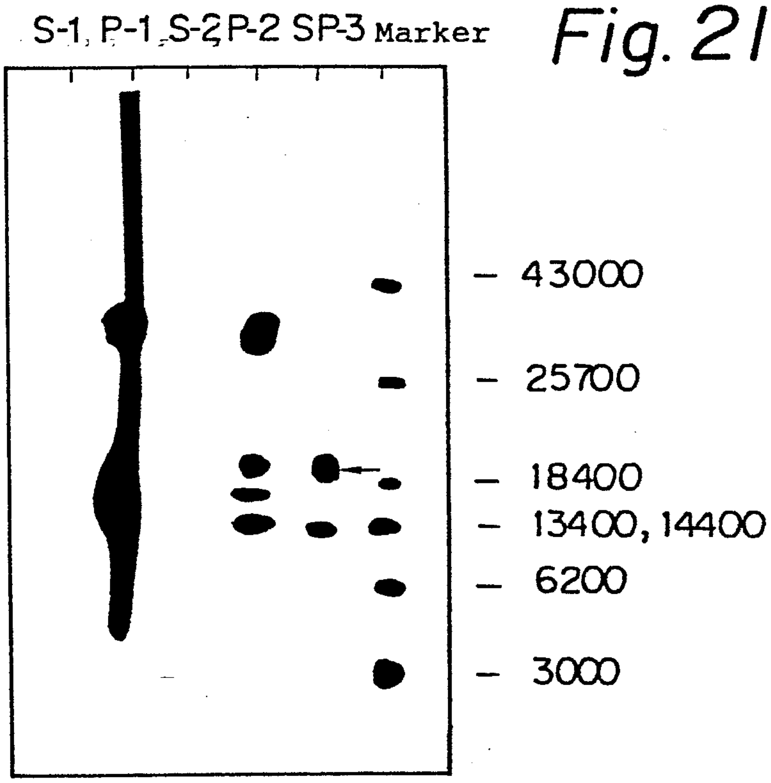

S-1, P-1, S-2, P-2, SP-3 Marker
43000
25700
18400
13400, 14400
6200
3000


*Fig. 21*

*Fig. 22*

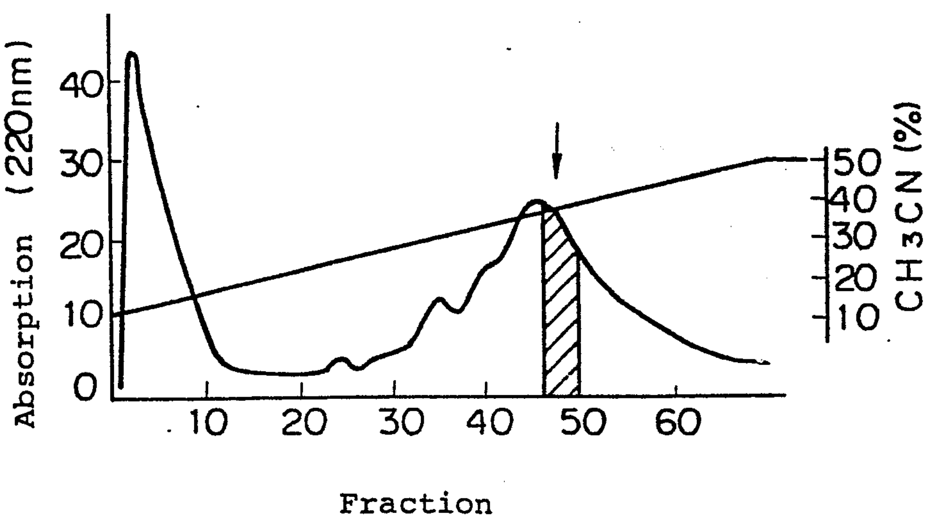

Absorption (220nm)
40
30
20
10
0
10
20
30
40
50
60
Fraction
CH3CN (%)
50
40
30
20
10

Fig. 23

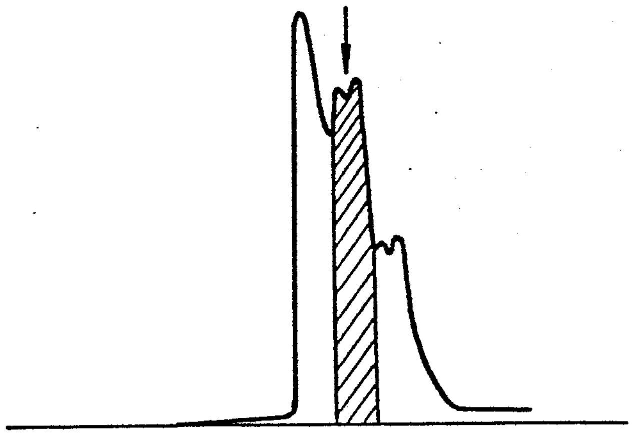

Fig. 24

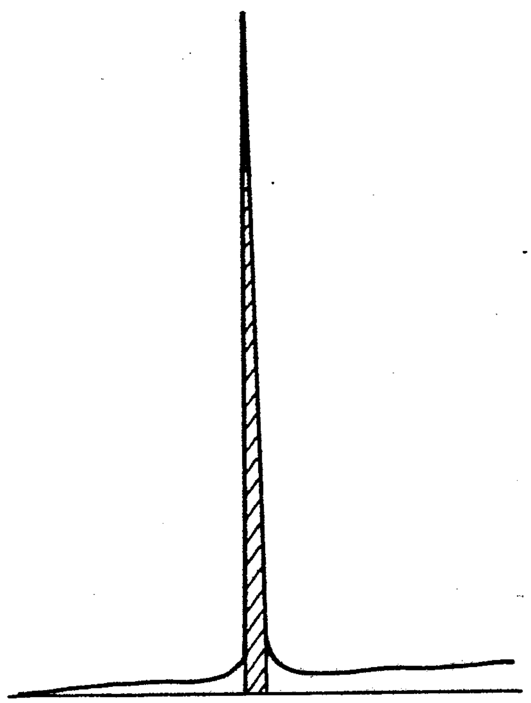

Fig. 25

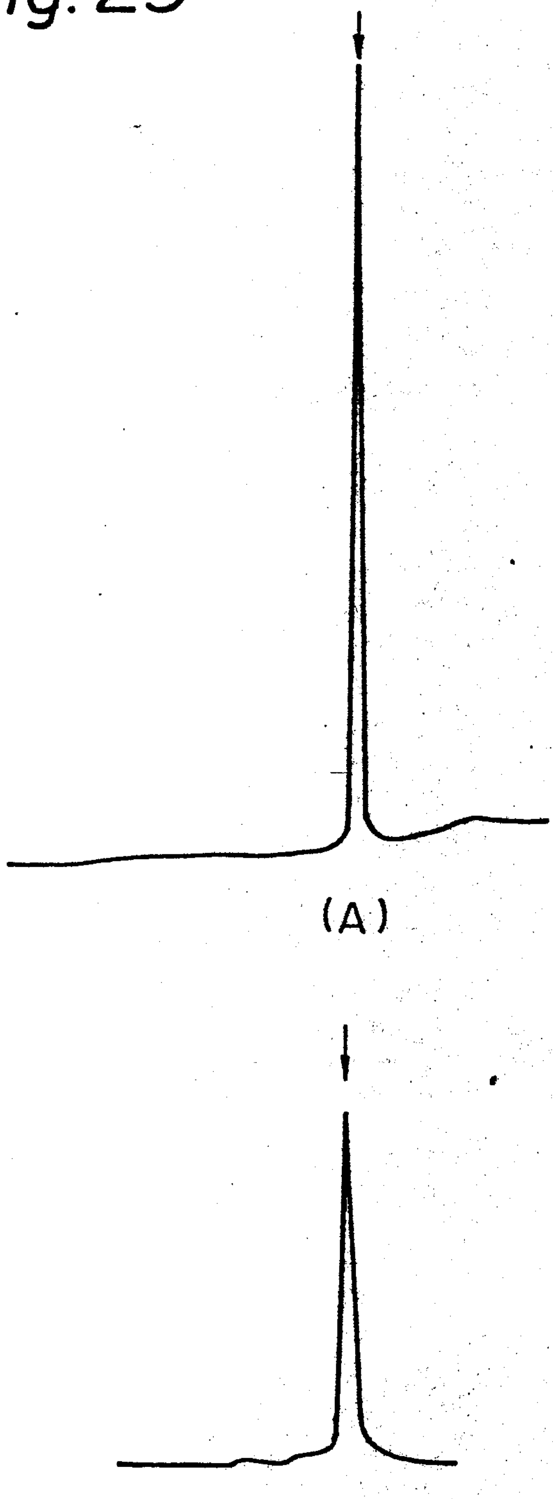

(A)

(B)

# INTERNATIONAL SEARCH REPORT



International Application No. PCT/JP85/00459

**I. CLASSIFICATION OF SUBJECT MATTER** (if several classification symbols apply, indicate all)[3]

According to International Patent Classification (IPC) or to both National Classification and IPC

Int.Cl[4] C12N15/00, C12N1/20, C07K7/36, C12P21/02
//(C12N1/20, C12R1:19) (C12P21/02, C12R1:19)

**II. FIELDS SEARCHED**

Minimum Documentation Searched[4]

| Classification System | Classification Symbols |
|---|---|
| IPC | C12N 15/00, C07K 7/36, C12P 21/02 |

Documentation Searched other than Minimum Documentation to the Extent that such Documents are Included in the Fields Searched[5]

**III. DOCUMENTS CONSIDERED TO BE RELEVANT**[14]

| Category* | Citation of Document,[16] with indication, where appropriate, of the relevant passages[17] | Relevant to Claim No.[18] |
|---|---|---|
| A | JP, A, 58-203953 (Suntory Limited) 28 November 1983 (28. 11. 83) & EP, A2, 95351 | 1-41 |
| A | JP, A, 59-501095 (Amgen) 28 June 1984 (28. 06. 84) & EP, A1, 107710 & WO, A1, 8304028 | 1-41 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search[2] | Date of Mailing of this International Search Report[2] |
|---|---|
| November 11, 1985 (11. 11. 85) | November 18, 1985 (18. 11. 85) |
| **International Searching Authority**[1] | **Signature of Authorized Officer**[20] |
| Japanese Patent Office | |